# EUROPÄISCHE PATENTANMELDUNG

(11) **EP 4 821 530 A2**
(43) Veröffentlichungstag der Anmeldung: **14.10.2026**
(21) Anmeldenummer: 26201512.6
(22) Anmeldetag: 26.06.2025
(51) Int. Cl.: A61M 16/06

(54) **ATEMMASKE ZUM VERSORGEN EINES PATIENTEN MIT ATEMGAS**

(30) Priorität: 30.07.2024 DE 102024121700
(62) Dokumentnummer(n) der früheren Anmeldung(en) nach Art. 76 EPÜ: 25185518.5 / 4 686 491
(71) Anmelder: Löwenstein Medical Technology S.A., 2557 Luxembourg (LU)
(72) Erfinder: Eifler, Martin, Gluckstadt (DE)
(74) Vertreter: Löwenstein Medical IP

(57) **Zusammenfassung**

Eine Atemmaske umfasst einen Maskenkörper (2), eine Kopfbänderung, ein erstes Koppelelement (7a) zum Koppeln der Kopfbänderung mit einer ersten Koppelaufnahme (3a) des Maskenkörpers und ein zweites Koppelelement zum Koppeln der Kopfbänderung mit einer zweiten Koppelaufnahme des Maskenkörpers. Jedes Koppelelement umfasst einen Koppelabschnitt (8) zum Koppeln des Koppelelements mit der jeweiligen Koppelaufnahme und eine Bandaufnahme (9a, 9b) zum Befestigen eines Bands der Kopfbänderung. Der Koppelabschnitt von mindestens einem der Koppelelemente ist mit der jeweiligen Koppelaufnahme zu einer Steckverbindung verbindbar. Ferner umfasst die Atemmaske ein zwischen einer Verriegelungsstellung und einer Entriegelungsstellung bewegbar gelagertes Verriegelungselement (18) und ein zwischen einer Ruhestellung und einer Betätigungsstellung bewegbar gelagertes Betätigungselement (19). Das Verriegelungselement ist ausgebildet, um die Steckverbindung in der Verriegelungsstellung zu verriegeln und in der Entriegelungsstellung zu entriegeln. Das Betätigungselement ist ausgebildet, um beim Bewegen in die Betätigungsstellung gegen das Verriegelungselement zu drücken, sodass das Verriegelungselement in die Entriegelungsstellung bewegt wird.

## Beschreibung

### Technisches Gebiet

Die Erfindung betrifft eine Atemmaske zum Versorgen eines Patienten mit Atemgas.

### Stand der Technik

Atemmasken, die als Schnittstelle zwischen einem Patienten und einem Beatmungsgerät eingesetzt werden, müssen hohen Anforderungen an Stabilität, Sicherheit und Komfort genügen und sollten gleichzeitig einfach in der Handhabung sein. Solche Atemmasken werden üblicherweise über ein oder mehrere Bänder am Kopf des Patienten fixiert. Insbesondere bei einer Heimbeatmung ist der Patient häufig selbst für das korrekte Anlegen und Fixieren der Atemmaske am Gesicht verantwortlich.

Manchen Patienten wie motorisch eingeschränkten oder älteren Personen oder Kindern kann das Anlegen der Atemmaske Schwierigkeiten bereiten. Allerdings sind eine korrekte Befestigung und ein damit einhergehender stabiler, möglichst dichter Sitz der Atemmaske eine Voraussetzung für eine sichere und effiziente Beatmung.

### Offenbarung der Erfindung

Eine Aufgabe der Erfindung kann darin gesehen werden, eine Atemmaske zur Verfügung zu stellen, die sich besonders einfach anlegen oder abnehmen lässt und/oder einen verbesserten Tragekomfort bietet.

Diese Aufgabe wird durch den Gegenstand des unabhängigen Anspruchs gelöst. Vorteilhafte Ausführungsformen sind in den abhängigen Ansprüchen, der nachfolgenden Beschreibung und den beigefügten Figuren dargelegt.

Offenbart wird eine Atemmaske zum Versorgen eines Patienten mit Atemgas, wobei die Atemmaske umfasst: einen Maskenkörper zum Anlegen an den Mund und/oder die Nase des Patienten, wobei der Maskenkörper eine erste Koppelaufnahme, eine zweite Koppelaufnahme und einen Schlauchanschluss zum Anschließen eines Beatmungsschlauchs umfasst; eine Kopfbänderung zum Fixieren des Maskenkörpers gegenüber dem Gesicht des Patienten, wobei die Kopfbänderung ein erstes Band und ein zweites Band zum Spannen um den Kopf des Patienten umfasst; ein erstes Koppelelement zum Koppeln der Kopfbänderung mit der ersten Koppelaufnahme; ein zweites Koppelelement zum Koppeln der Kopfbänderung mit der zweiten Koppelaufnahme. Jedes Koppelelement umfasst einen Koppelabschnitt zum Koppeln des Koppelelements mit der jeweiligen Koppelaufnahme, eine erste Bandaufnahme zum Befestigen des ersten Bands und eine zweite Bandaufnahme zum Befestigen des zweiten Bands. Jedes Koppelelement ist so ausgebildet, dass - wenn der Maskenkörper mittels der Kopfbänderung gegenüber dem Gesicht des Patienten fixiert ist - die erste Bandaufnahme in Richtung einer Sagittalachse des Patienten betrachtet zumindest teilweise zwischen dem Kinn und einem Ohr des Patienten und in Richtung einer zur Sagittalachse orthogonalen Longitudinalachse des Patienten betrachtet zumindest teilweise zwischen dem Kinn und der Schläfenregion des Patienten angeordnet ist und die zweite Bandaufnahme zumindest teilweise gegenüber der Schläfenregion des Patienten angeordnet ist.

Eine solche Atemmaske lässt sich aufgrund der speziellen Anordnung der Bandaufnahmen in Bezug auf das Kinn, die Ohren und die Schläfenregion des Patienten besonders einfach anlegen oder abnehmen und besonders komfortabel tragen. Zudem gewährleistet die Atemmaske aufgrund der daraus resultierenden speziellen Hebelverhältnisse einen sicheren, ausreichend dichten Sitz des Maskenkörpers. Insbesondere ermöglicht die Atemmaske eine Positionierung der Koppelelemente und/oder der Bänder (oder zumindest eines größeren Teils davon) außerhalb des Gesichts- oder Blickfelds des Patienten, was beispielsweise Brillenträgern zugutekommt. Ein weiterer Vorteil ist, dass die Atemmaske ohne eine möglicherweise störende Stirnstütze auskommt, was die Handhabung der Atemmaske weiter vereinfacht.

Offenbart wird ferner eine Atemmaske zum Versorgen eines Patienten mit Atemgas, wobei die Atemmaske umfasst: einen Maskenkörper zum Anlegen an den Mund und/oder die Nase des Patienten, wobei der Maskenkörper eine erste Koppelaufnahme, eine zweite Koppelaufnahme und einen Schlauchanschluss zum Anschließen eines Beatmungsschlauchs umfasst; eine Kopfbänderung zum Fixieren des Maskenkörpers gegenüber dem Gesicht des Patienten; ein erstes Koppelelement zum Koppeln der Kopfbänderung mit der ersten Koppelaufnahme; ein zweites Koppelelement zum Koppeln der Kopfbänderung mit der zweiten Koppelaufnahme. Jedes Koppelelement umfasst einen Koppelabschnitt zum Koppeln des Koppelelements mit der jeweiligen Koppelaufnahme und eine oder mehrere Bandaufnahmen zum Befestigen eines oder mehrerer Bänder der Kopfbänderung. Der Koppelabschnitt von mindestens einem (oder jedem) der beiden Koppelelemente ist mit der jeweiligen Koppelaufnahme zu einer Steckverbindung verbindbar. Ferner umfasst die Atemmaske ein am Maskenkörper und/oder am jeweiligen Koppelelement zwischen einer Verriegelungsstellung und einer Entriegelungsstellung bewegbar gelagertes Verriegelungselement, das ausgebildet ist, um die Steckverbindung in der Verriegelungsstellung zu verriegeln, sodass ein Verlagern des Koppelabschnitts in einer Trennrichtung weg von der jeweiligen Koppelaufnahme nicht möglich ist, und in der Entriegelungsstellung zu entriegeln, sodass das Verlagern des Koppelabschnitts in der Trennrichtung möglich ist. Darüber hinaus umfasst die Atemmaske ein am Maskenkörper und/oder am jeweiligen Koppelelement zwischen einer Ruhestellung und einer Betätigungsstellung bewegbar gelagertes Betätigungselement zum Betätigen des Verriegelungselements, wenn die Steckverbindung verriegelt ist. Das Betätigungselement ist ausgebildet, um beim Bewegen in die Betätigungsstellung gegen das Verriegelungselement zu drücken, sodass das Verriegelungselement in die Entriegelungsstellung bewegt wird.

Eine solche Atemmaske lässt sich aufgrund der Steckverbindung besonders einfach anlegen oder abnehmen und gewährleistet aufgrund der Verriegelung einen sicheren Sitz des Maskenkörpers. Zudem ermöglicht die indirekte Betätigung des Verriegelungselements über das Betätigungselement eine besonders ergonomische Ausgestaltung des Betätigungselements unabhängig von den konstruktiven Eigenheiten des Verriegelungselements. Dies kann den Bedienkomfort gegenüber einer Ausführungsform ohne ein derartiges Betätigungselement signifikant verbessern.

Des Weiteren wird eine Atemmaske zum Versorgen eines Patienten mit Atemgas offenbart, wobei die Atemmaske umfasst: einen Maskenkörper zum Anlegen an den Mund und/oder die Nase des Patienten, wobei der Maskenkörper eine erste Koppelaufnahme, eine zweite Koppelaufnahme und einen Schlauchanschluss zum Anschließen eines Beatmungsschlauchs umfasst; eine Kopfbänderung zum Fixieren des Maskenkörpers gegenüber dem Gesicht des Patienten; ein erstes Koppelelement zum Koppeln der Kopfbänderung mit der ersten Koppelaufnahme; ein zweites Koppelelement zum Koppeln der Kopfbänderung mit der zweiten Koppelaufnahme. Jedes Koppelelement umfasst einen Koppelabschnitt zum Koppeln des Koppelelements mit der jeweiligen Koppelaufnahme und eine Bandaufnahme zum Befestigen eines Bands der Kopfbänderung. Der Koppelabschnitt von mindestens einem (oder jedem) der beiden Koppelelemente umfasst einen hakenförmigen Hakenabschnitt, der mit der jeweiligen Koppelaufnahme verhakbar ist, um eine Verhakung zum Befestigen der Kopfbänderung am Maskenkörper zu bilden. Des Weiteren umfasst der Koppelabschnitt einen flügelförmigen Betätigungsabschnitt, der mit dem Hakenabschnitt fest verbunden ist und so angeordnet ist, dass der Betätigungsabschnitt - wenn der Maskenkörper mittels der Kopfbänderung gegenüber dem Gesicht des Patienten fixiert ist (und der Hakenabschnitt mit der jeweiligen Koppelaufnahme verhakt ist) - derart vom übrigen Koppelabschnitt absteht, dass der Betätigungsabschnitt mit mindestens einem Finger in einer Schwenkrichtung weg von der jeweiligen Koppelaufnahme geschwenkt werden kann. Die Verhakung ist so ausgebildet, dass der Hakenabschnitt - wenn der Maskenkörper mittels der Kopfbänderung gegenüber dem Gesicht des Patienten fixiert ist - durch eine von der Kopfbänderung ausgeübte Zugkraft gegen die jeweilige Koppelaufnahme gedrückt wird, um ein ungewolltes Lösen der Verhakung zu verhindern, und durch Schwenken des Betätigungsabschnitts in der Schwenkrichtung aus der jeweiligen Koppelaufnahme herausbewegbar ist, um ein kontrolliertes Lösen der Verhakung zu ermöglichen.

Eine derartige Verhakung stellt eine brauchbare Alternative zur vorstehend beschriebenen Steckverbindung dar.

Unter "Atemmaske" kann beispielsweise eine Mundmaske, eine Nasenmaske oder eine Kombination aus einer Mund- und einer Nasenmaske, auch Full-Face-Maske genannt, verstanden werden. Die Atemmaske kann zur Verwendung mit einem Beatmungsgerät geeignet sein. Unter "Beatmungsgerät" kann ein Gerät zum invasiven und/oder nicht invasiven Beatmen des Patienten und/oder zum Unterstützen des Patienten beim Husten verstanden werden. Das Beatmungsgerät kann auch ein Anästhesiegerät sein.

Unter "Sagittalachse" kann eine ventrodorsale Achse, auch Pfeilachse genannt, verstanden werden. Unter "Longitudinalachse" kann eine (zur ventrodorsalen Achse orthogonale) kraniokaudale Achse verstanden werden. Sofern der Patient aufrecht steht, kann die Sagittalachse mit einer Horizontalen bzw. kann die Longitudinalachse mit einer Vertikalen übereinstimmen.

Unter "Band" kann allgemein ein länglicher Abschnitt der Kopfbänderung zum Ausüben einer geeigneten Zugkraft auf die Koppelelemente verstanden werden. Ein solches Band kann besonders elastisch ausgeführt sein. Beispielsweise kann das Band als ein Gummi- und/oder Textilband ausgeführt sein. Durch entsprechendes Verstellen des Bandes oder der Bänder ist es möglich, die Position und/oder Ausrichtung eines jeden Koppelelements, insbesondere der Bandaufnahme(n) und des Koppelabschnitts, relativ zum Kopf des Patienten zu verändern. Die Kopplung des Maskenkörpers mit den Koppelelementen kann derart sein, dass sich eine Verstellung des Bandes oder der Bänder in entsprechender Weise auf die Position und/oder Ausrichtung des Maskenkörpers relativ zum Gesicht des Patienten auswirkt. Dazu können die Koppelelemente beispielsweise entsprechend verwindungssteif ausgeführt und/oder entsprechend starr mit dem Maskenkörper koppelbar sein.

Bei dem ersten Band und dem zweiten Band kann es sich um einzelne Bänder oder einen Bandverbund handeln. Die Bänder des Bandverbunds können direkt und/oder indirekt (beispielsweise über einen oder mehrere zusätzliche Abschnitte der Kopfbänderung) miteinander verbunden sein. Zweckmäßigerweise können das erste Band und das zweite Band unabhängig voneinander in ihrer Länge und/oder Zugkraft verstellbar ausgebildet sein.

Die Kopfbänderung kann und/oder das erste Band und das zweite Band können zusätzlich zumindest stellenweise mit einem im Vergleich zu Gummi oder Textil besonders steifen Material wie beispielsweise einem Thermo- oder Duroplast verstärkt sein. Die Kopfbänderung kann auch mehr als zwei Bänder umfassen.

Es ist möglich, dass der Endabschnitt eines jeden Bands durch die jeweilige Bandaufnahme hindurchgeführt und mit dem restlichen Abschnitt des Bands und/oder mit dem restlichen Abschnitt der Kopfbänderung zu einer Schlaufe verbunden werden kann. Die Verbindung kann beispielsweise eine Klettverbindung sein und/oder über Schnallen, Knöpfe, Haken, Ösen, Riemen oder Knoten realisiert sein.

Der Maskenkörper kann so ausgebildet sein, dass er den Mund und/oder die Nase (oder zumindest die Nasenlöcher) des Patienten luftdicht umschließt, wenn er mittels der Kopfbänderung gegenüber dem Gesicht des Patienten fixiert ist. Dabei kann der Maskenkörper zusammen mit dem umschlossenen Teil des Gesichts einen Atemgasraum begrenzen, der über den Schlauchanschluss und den daran angeschlossenen Beatmungsschlauch mit Atemgas gefüllt oder entleert werden kann. Der Maskenkörper kann bei Verwendung der Atemmaske mittelbar (beispielsweise über einen Maskenwulst und/oder ein Maskenkissen) und/oder unmittelbar am Gesicht des Patienten anliegen.

Die Koppelaufnahmen können beidseitig am Maskenkörper angeordnet sein. So kann der Maskenkörper eine Koppelaufnahme an einer linken Seite und eine weitere Koppelaufnahme an einer rechten Seite aufweisen (aus der Sicht des die Atemmaske tragenden Patienten betrachtet).

Die Koppelelemente in Kombination mit der Kopfbänderung ermöglichen es, einen Anpressdruck, mit dem der Maskenkörper gegen das Gesicht gedrückt wird, und/oder eine Neigung des Maskenkörpers in Bezug auf die Sagittalachse und/oder die Longitudinalachse und/oder eine zur Sagittalachse und zur Longitudinalachse orthogonale Transversalachse des Körpers des Patienten (auch Querachse genannt) an die individuellen Gesichtskonturen des Patienten anzupassen. Somit können unangenehme oder sogar schmerzhafte Druckstellen vermieden werden. Dazu kann jedes Koppelelement (oder zumindest ein Teil davon) als ein Hebel zum zusätzlichen Beaufschlagen des Maskenkörpers mit einem bestimmten Drehmoment ausgebildet sein. Dieses Drehmoment, genauer dessen Betrag und Richtung, kann von den durch die Kopfbänderung auf die Bandaufnahmen ausgeübten Zugkräften, genauer von deren jeweiligem Betrag und jeweiliger Richtung, abhängen. Die Kopfbänderung ist somit nicht direkt, sondern indirekt über die Koppelelemente mit dem Maskenkörper verbunden. Beispielsweise kann bei Verwendung der Atemmaske das erste Koppelelement der linken Gesichtshälfte und das zweite Koppelelement der rechten Gesichtshälfte zugewandt sein.

Jedes Koppelelement kann beispielsweise so ausgebildet sein, dass ein erster Abstand zwischen der ersten Bandaufnahme und der zweiten Bandaufnahme (genauer zwischen einem ersten Kraftangriffspunkt, an dem eine erste Zugkraft angreift, die von dem an der ersten Bandaufnahme befestigten ersten Band aufgebracht wird, und einem zweiten Kraftangriffspunkt, an dem eine zweite Zugkraft angreift, die von dem an der zweiten Bandaufnahme befestigten zweiten Band aufgebracht wird) und ein zweiter Abstand zwischen der ersten Bandaufnahme und dem Koppelabschnitt (genauer zwischen dem ersten Kraftangriffspunkt und einem dritten Kraftangriffspunkt, an dem eine Reaktionskraft angreift, die von der mit dem Koppelabschnitt gekoppelten Koppelaufnahme des Maskenkörpers aufgebracht wird) miteinander übereinstimmen oder um höchstens 20 Prozent, vorzugsweise höchstens 10 Prozent, voneinander abweichen. Alternativ kann der erste Abstand auch deutlich (beispielsweise um mehr als 20 Prozent oder mindestens 50 Prozent) größer als der zweite Abstand sein (oder umgekehrt). Unter "erster Abstand" kann in diesem Zusammenhang eine Länge eines ersten Hebelarms verstanden werden. In entsprechender Weise kann unter "zweiter Abstand" eine Länge eines zweiten Hebelarms verstanden werden. Ein von den beiden Hebelarmen eingeschlossener Winkel kann beispielsweise zwischen 60 und 120 Grad, zwischen 80 und 100 Grad oder zwischen 45 und 90 Grad, insbesondere 70 Grad, betragen.

Zweckmäßigerweise können die Koppelelemente aus einem im Vergleich zu Gummi oder Textil besonders steifen Material wie beispielsweise einem Thermo- oder Duroplast, einem Metall oder einer Kombination von mindestens zwei dieser Beispiele ausgebildet sein.

Die Koppelelemente können baugleich ausgebildet sein. Alternativ können die Koppelelemente unterschiedlich ausgeformt sein, insbesondere derart, dass sie jeweils nur mit genau einer der Koppelaufnahmen gekoppelt werden können, was das Anlegen der Atemmaske weiter vereinfachen kann. Jedes Koppelelement kann auch mehr als zwei Bandaufnahmen und/oder mehr als einen Koppelabschnitt umfassen.

Der Maskenkörper und die beiden Koppelelemente können auch einstückig und/oder aus dem gleichen Material, beispielsweise einem thermoplastischen Kunststoff, ausgebildet sein.

Der Kopf des Patienten kann auch dessen Nacken umfassen.

Im Folgenden werden verschiedene Ausführungsformen beschrieben. Diese Ausführungsformen sind nicht als Beschränkung des Umfangs der Offenbarung zu verstehen.

Gemäß einer Ausführungsform kann jedes Koppelelement so ausgebildet sein, dass - wenn der Maskenkörper mittels der Kopfbänderung gegenüber dem Gesicht des Patienten fixiert ist (d. h. bei Verwendung der Atemmaske) - die erste Bandaufnahme zumindest teilweise gegenüber einem Teil des Unterkiefers des Patienten, insbesondere gegenüber einem Unterkieferast, und/oder außerhalb der Wangenregion des Patienten angeordnet ist. Dies ermöglicht es, das erste Band unterhalb der Ohren über die Nackenregion und/oder das Hinterhauptbein des Patienten zu spannen. Somit kann die erste Bandaufnahme bzw. das erste Band zumindest größtenteils außerhalb des Gesichts- oder Blickfelds des Patienten positioniert werden.

Gemäß einer Ausführungsform kann jedes Koppelelement so ausgebildet sein, dass - wenn der Maskenkörper mittels der Kopfbänderung gegenüber dem Gesicht des Patienten fixiert ist - der mit der jeweiligen Koppelaufnahme gekoppelte Koppelabschnitt in Richtung der Longitudinalachse betrachtet zumindest teilweise zwischen der ersten Bandaufnahme und der zweiten Bandaufnahme angeordnet ist. Zusätzlich oder alternativ kann die zweite Bandaufnahme in Richtung der Sagittalachse betrachtet zumindest teilweise zwischen der ersten Bandaufnahme und dem mit der jeweiligen Koppelaufnahme gekoppelten Koppelabschnitt angeordnet sein.

Es ist möglich, dass - in Richtung der Longitudinalachse betrachtet - die erste Bandaufnahme zumindest teilweise unterhalb des mit der jeweiligen Koppelaufnahme gekoppelten Koppelabschnitts und/oder die zweite Bandaufnahme zumindest teilweise oberhalb des mit der jeweiligen Koppelaufnahme gekoppelten Koppelabschnitts angeordnet ist.

Zudem kann die zweite Bandaufnahme in Richtung der Longitudinalachse betrachtet zumindest teilweise oberhalb des Maskenkörpers und/oder oberhalb der Ohren des Patienten und/oder oberhalb der Augen des Patienten und/oder in Richtung der Sagittalachse betrachtet zumindest teilweise zwischen dem Auge und dem Ohr der jeweiligen Gesichtshälfte des Patienten angeordnet sein.

Dies ermöglicht es, das zweite Band oberhalb der Ohren und/oder über den Hinterkopf, beispielsweise über das Scheitelbein, zu spannen. Somit kann die zweite Bandaufnahme bzw. das zweite Band zumindest größtenteils außerhalb des Gesichts- oder Blickfelds des Patienten positioniert werden.

Darüber hinaus kann die zweite Bandaufnahme in Richtung der Sagittalachse betrachtet (deutlich) näher am Maskenkörper als die erste Bandaufnahme angeordnet sein (oder umgekehrt). Beispielsweise kann ein Abstand der ersten Bandaufnahme zum Maskenkörper in Richtung der Sagittalachse um mindestens 10 Prozent, mindestens 20 Prozent oder mindestens 40 Prozent von einem entsprechenden Abstand der zweiten Bandaufnahme abweichen.

Möglich ist auch, dass die erste Bandaufnahme und die zweite Bandaufnahme in Richtung der Longitudinalachse betrachtet zumindest teilweise einander überlappen.

Gemäß einer Ausführungsform kann - wenn der Maskenkörper mittels der Kopfbänderung gegenüber dem Gesicht des Patienten fixiert ist - das erste Band eine erste Zugkraft auf die erste Bandaufnahme eines jeden Koppelelements ausüben und das zweite Band eine zweite Zugkraft auf die zweite Bandaufnahme eines jeden Koppelelements ausüben. In diesem Fall ist es möglich, dass:
- eine in Richtung der Sagittalachse wirkende Komponente der ersten Zugkraft (deutlich) größer als, vorzugsweise mindestens doppelt so groß wie eine in Richtung der Longitudinalachse wirkende Komponente der ersten Zugkraft ist und/oder
- eine in Richtung der Sagittalachse wirkende Komponente der zweiten Zugkraft (deutlich) größer als, vorzugsweise mindestens doppelt so groß wie eine in Richtung der Longitudinalachse wirkende Komponente der zweiten Zugkraft ist und/oder
- die erste Zugkraft und die zweite Zugkraft innerhalb eines vorgegebenen Toleranzbereichs parallel zueinander ausgerichtet sind und/oder
- die erste Zugkraft zur Nackenregion und/oder zum Hinterhauptbein des Patienten gerichtet ist und/oder
- die zweite Zugkraft zu einem Kopfabschnitt außerhalb der Nackenregion und/oder des Hinterhauptbeins des Patienten, insbesondere zum Scheitelbein des Patienten, gerichtet ist.

Beispielsweise kann die sagittale Komponente der ersten bzw. zweiten Zugkraft mindestens 120 %, mindestens 200 % oder sogar mindestens 300 % der jeweiligen longitudinalen Komponente betragen. In manchen Fällen ist es möglich, dass die longitudinale Komponente der ersten und/oder zweiten Zugkraft gleich null oder vernachlässigbar gering im Vergleich zur jeweiligen sagittalen Komponente ist. Anders ausgedrückt kann die erste und/oder zweite Zugkraft in manchen Fällen ausschließlich in Richtung der Sagittalachse wirken.

Der vorgegebene Toleranzbereich kann beispielsweise einer zulässigen Winkelabweichung von höchstens plus/minus 10 Grad, vorzugsweise höchstens plus/minus 5 Grad, zwischen den Wirkungslinien der beiden Zugkräfte entsprechen.

Gemäß einer Ausführungsform kann jedes Koppelelement einen ersten Arm und einen zweiten Arm umfassen. Der erste Arm und der zweite Arm können derart in einem Übergangsabschnitt miteinander verbunden sein, dass sie eine L- oder V-Form bilden. Dabei kann die erste Bandaufnahme zumindest teilweise im Übergangsabschnitt angeordnet sein und/oder die zweite Bandaufnahme an einem dem Übergangsabschnitt abgewandten Ende des zweiten Arms angeordnet sein und/oder der Koppelabschnitt an einem dem Übergangsabschnitt abgewandten Ende des ersten Arms angeordnet sein. Dies ermöglicht eine besonders kompakte und dennoch ausreichend stabile Ausführung der Koppelelemente. Alternativ können der erste Arm und der zweite Arm auch zu einer T-Form miteinander verbunden sein.

Gemäß einer Ausführungsform kann jede erste Bandaufnahme und/oder kann jede zweite Bandaufnahme eine im Material des jeweiligen Koppelelements ausgebildete Aufnahmeöffnung zum Einführen des jeweiligen Bands umfassen. Die Aufnahmeöffnung kann zumindest teilweise durch einen in einer Umfangsrichtung der Aufnahmeöffnung gekrümmten (beispielsweise kreisbogenförmigen) Randabschnitt begrenzt sein. Das an der jeweiligen Bandaufnahme befestigte Band kann den Randabschnitt zumindest teilweise umschlingen. Beispielsweise kann das Band eine um den Randabschnitt herumgeführte Schlaufe bilden, wenn der Maskenkörper mittels der Kopfbänderung gegenüber dem Gesicht des Patienten fixiert ist. Eine Ausrichtung einer Längsachse des jeweiligen Bands relativ zur Sagittalachse kann durch Verschieben des den Randabschnitt umschlingenden Teils des jeweiligen Bands entlang des Randabschnitts veränderbar sein. Auf diese Weise kann ein von der Sagittalachse und der Längsachse des Bands eingeschlossener Winkel sehr einfach und komfortabel an die individuellen Gesichts- oder Kopfkonturen des Patienten angepasst werden.

Gemäß einer Ausführungsform kann der Koppelabschnitt von mindestens einem (oder jedem) der beiden Koppelelemente mit der jeweiligen Koppelaufnahme zu einer (wiederlösbaren) Steckverbindung verbindbar sein. In diesem Fall kann die Atemmaske ferner ein am Maskenkörper (beispielsweise an der Koppelaufnahme) und/oder am jeweiligen Koppelelement (beispielsweise am Koppelabschnitt) zwischen einer Verriegelungsstellung und einer Entriegelungsstellung bewegbar gelagertes Verriegelungselement umfassen. Das Verriegelungselement kann ausgebildet sein, um die Steckverbindung in der Verriegelungsstellung zu verriegeln, sodass ein Verlagern des Koppelabschnitts in einer Trennrichtung weg von der jeweiligen Koppelaufnahme nicht möglich ist, und in der Entriegelungsstellung zu entriegeln, sodass das Verlagern des Koppelabschnitts in der Trennrichtung (zumindest in begrenztem Maß) möglich ist.

Anders ausgedrückt kann der Koppelabschnitt mit der jeweiligen Koppelaufnahme mittels eines Rastmechanismus verrastbar sein. Der Rastmechanismus kann ausgebildet sein, um den Koppelabschnitt in einer vorgegebenen Position oder in einer von mehreren möglichen vorgegebenen Positionen mit der Koppelaufnahme mittels Federkraft zu verrasten. Somit können die Koppelelemente auf sehr einfache und komfortable Weise mit dem Maskenkörper gekoppelt und wieder davon gelöst werden.

Gemäß einer Ausführungsform kann die Atemmaske ferner ein am Maskenkörper und/oder am jeweiligen Koppelelement zwischen einer Ruhestellung und einer Betätigungsstellung bewegbar gelagertes Betätigungselement umfassen. Das Betätigungselement kann geeignet sein, um das Verriegelungselement zu betätigen, wenn die Steckverbindung verriegelt ist. Das Betätigungselement kann ausgebildet sein, um beim Bewegen in die Betätigungsstellung gegen das Verriegelungselement zu drücken, sodass das Verriegelungselement in die Entriegelungsstellung bewegt wird. Eine derartige indirekte Betätigung des Verriegelungselements ermöglicht eine besonders ergonomische Ausgestaltung des Betätigungselements unabhängig von den konstruktiven Eigenheiten des Verriegelungselements. Dies kann den Bedienkomfort weiter verbessern.

Gemäß einer Ausführungsform kann das Betätigungselement ausgebildet sein, um einen Verlagerungsweg des Koppelabschnitts beim Verlagern in der Trennrichtung in der Betätigungsstellung zu begrenzen, sodass der Koppelabschnitt nur bis in eine Zwischenstellung relativ zur jeweiligen Koppelaufnahme verlagert werden kann, und in der Ruhestellung nicht zu begrenzen, sodass der Koppelabschnitt über die Zwischenstellung hinaus verlagert werden kann. Dabei kann die Steckverbindung durch Verlagern des Koppelabschnitts über die Zwischenstellung hinaus trennbar sein. Anders ausgedrückt können der Koppelabschnitt und die Koppelaufnahme der Steckverbindung nur dadurch (vollständig) voneinander getrennt werden, dass der Koppelabschnitt über die Zwischenstellung hinaus verlagert wird. Dies hat die Wirkung, dass die Steckverbindung nicht sofort beim erstmaligen Betätigen (beispielsweise Drücken) des Betätigungselements getrennt wird. Unter Umständen könnte der Koppelabschnitt dabei infolge der durch die Kopfbänderung ausgeübten Zugkräfte in unkontrollierter Weise von der Koppelaufnahme weggeschleudert werden. Dies kann vermieden werden, indem die Steckverbindung erst dann (vollständig) getrennt wird, wenn das Betätigungselement nach dem Bewegen in die Betätigungsstellung wieder in die Ruhestellung zurückbewegt wird. Somit wird eine kontrollierte Trennung der Steckverbindung ermöglicht.

Gemäß einer Ausführungsform kann das Verriegelungselement durch Verlagern des Koppelabschnitts bis in die Zwischenstellung gegenüber einer Anschlagkante des Maskenkörpers (beispielsweise der Koppelaufnahme) und/oder des jeweiligen Koppelelements (beispielsweise des Koppelabschnitts) positionierbar sein. Die Anschlagkante kann ausgebildet sein, um das gegenüber der Anschlagkante positionierte Verriegelungselement daran zu hindern, in die Verriegelungsstellung zurückzukehren.

Dazu kann das gegenüber der Anschlagkante positionierte Verriegelungselement in geeigneter Weise an der Anschlagkante anliegen, d. h. diese (unmittelbar) berühren. Das Verlagern des Koppelabschnitts bis in die Zwischenstellung beim Entriegeln der Steckverbindung kann beispielsweise allein aufgrund der durch die Kopfbänderung ausgeübten Zugkräfte erfolgen. Dadurch, dass das Verriegelungselement in der Zwischenstellung in geeigneter Weise mechanisch blockiert wird, kann ein ungewolltes erneutes Verriegeln der Steckverbindung beim Bewegen des Betätigungselements zurück in die Ruhestellung verhindert werden.

Gemäß einer Ausführungsform kann das Betätigungselement in der Betätigungsstellung in eine vorzugsweise keilförmige Vertiefung im Maskenkörper (beispielsweise in der Koppelaufnahme) und/oder im jeweiligen Koppelelement (beispielsweise im Koppelabschnitt) eingreifen. Das Betätigungselement kann durch Zurückbewegen in die Ruhestellung aus der Vertiefung herausbewegbar sein. Die Vertiefung kann in einer Richtung quer zur Trennrichtung durch zwei schräg zueinander ausgerichtete Begrenzungskanten begrenzt sein. Die beiden Begrenzungskanten können derart schräg zueinander ausgerichtet sein, dass sie in einer der Trennrichtung entgegengesetzten Richtung betrachtet aufeinander zulaufen. Das Betätigungselement kann so ausgebildet sein, dass ein in die Vertiefung eingreifender Abschnitt des Betätigungselements beim Verlagern des Koppelabschnitts in der Trennrichtung auf die Begrenzungskanten zubewegt wird und - wenn der Koppelabschnitt die Zwischenstellung erreicht - beidseitig an den Begrenzungskanten anschlägt, sodass ein weiteres Verlagern des Koppelabschnitts über die Zwischenstellung hinaus nicht möglich ist.

Gemäß einer Ausführungsform kann das Betätigungselement als eine Betätigungslasche ausgebildet sein. Die Betätigungslasche kann an einem ihrer Enden mit dem Maskenkörper (beispielsweise mit der Koppelaufnahme) und/oder mit dem jeweiligen Koppelelement (beispielsweise mit dem Koppelabschnitt) verbunden sein. Die Betätigungslasche kann durch elastisches Verbiegen ihres anderen, beispielsweise unverbundenen Endes in die Betätigungsstellung bewegbar sein, sodass auf die Betätigungslasche in der Betätigungsstellung eine Federkraft zum Zurückstellen der Betätigungslasche in die Ruhestellung einwirkt. Dies hat die Wirkung, dass sich das Betätigungselement beim Loslassen von allein in die Ruhestellung zurückbewegt, sofern es nicht anderweitig daran gehindert wird.

Gemäß einer Ausführungsform kann das Verriegelungselement als eine Verriegelungslasche ausgebildet sein. Die Verriegelungslasche kann an einem ihrer Enden mit dem Maskenkörper (beispielsweise mit der Koppelaufnahme) und/oder mit dem jeweiligen Koppelelement (beispielsweise mit dem Koppelabschnitt) verbunden sein. Die Verriegelungslasche kann durch elastisches Verbiegen ihres anderen, beispielsweise unverbundenen Endes in die Entriegelungsstellung bewegbar sein, sodass auf die Verriegelungslasche in der Entriegelungsstellung eine Federkraft zum Zurückstellen der Verriegelungslasche in die Verriegelungsstellung einwirkt. Dies hat die Wirkung, dass sich das Verriegelungselement beim Loslassen von allein in die Verriegelungsstellung zurückbewegt, sofern es nicht anderweitig daran gehindert wird.

Unter "Lasche" wie in "Verriegelungslasche" oder "Betätigungslasche" kann beispielsweise ein platten- oder fingerförmiges Element verstanden werden.

Gemäß einer Ausführungsform kann der Koppelabschnitt von mindestens einem (oder jedem) der beiden Koppelelemente einen hakenförmigen Hakenabschnitt umfassen, der mit der jeweiligen Koppelaufnahme verhakbar ist, um eine Verhakung zum Befestigen der Kopfbänderung am Maskenkörper zu bilden. Des Weiteren kann der Koppelabschnitt einen flügelförmigen Betätigungsabschnitt umfassen, der mit dem Hakenabschnitt fest verbunden ist und so angeordnet ist, dass der Betätigungsabschnitt - wenn der Maskenkörper mittels der Kopfbänderung gegenüber dem Gesicht des Patienten fixiert ist (und der Hakenabschnitt mit der jeweiligen Koppelaufnahme verhakt ist) - derart vom übrigen Koppelabschnitt absteht, dass der Betätigungsabschnitt mit mindestens einem Finger in einer Schwenkrichtung weg von der jeweiligen Koppelaufnahme geschwenkt werden kann. Die Verhakung kann so ausgebildet sein, dass der Hakenabschnitt - wenn der Maskenkörper mittels der Kopfbänderung gegenüber dem Gesicht des Patienten fixiert ist - durch eine von der Kopfbänderung ausgeübte Zugkraft gegen die jeweilige Koppelaufnahme gedrückt wird, um ein ungewolltes Lösen der Verhakung zu verhindern, und durch Schwenken des Betätigungsabschnitts in der Schwenkrichtung aus der jeweiligen Koppelaufnahme herausbewegbar ist, um ein kontrolliertes Lösen der Verhakung zu ermöglichen.

Gemäß einer Ausführungsform kann das jeweilige Koppelelement in dessen Längsrichtung betrachtet in mindestens einen ersten Längsabschnitt, einen zweiten Längsabschnitt und einen dritten Längsabschnitt unterteilt sein. Der zweite Längsabschnitt kann zwischen dem ersten Längsabschnitt und dem dritten Längsabschnitt angeordnet sein und/oder unmittelbar in den ersten Längsabschnitt und/oder unmittelbar in den dritten Längsabschnitt übergehen. Der erste Längsabschnitt kann in Bezug auf eine Längsachse des jeweiligen Koppelelements quer, schräg oder gerade von einem (dem dritten Längsabschnitt abgewandten) Ende des zweiten Längsabschnitts abstehen und/oder den Betätigungsabschnitt umfassen. Beispielsweise kann der erste Längsabschnitt als der Betätigungsabschnitt ausgebildet sein. Der zweite Längsabschnitt kann hingegen den Hakenabschnitt umfassen. Beispielsweise kann der zweite Längsabschnitt als der Hakenabschnitt ausgebildet sein. Bei dem dritten Längsabschnitt kann es sich um einen übrigen Längsabschnitt des jeweiligen Koppelelements handeln. Es ist möglich, dass der zweite Längsabschnitt über einen vierten Längsabschnitt, der einen nachstehend näher beschriebenen Biegeabschnitt umfassen kann oder als ein nachstehend näher beschriebener Biegeabschnitt ausgebildet sein kann, mit dem dritten Längsabschnitt verbunden ist.

Gemäß einer Ausführungsform kann der Koppelabschnitt des jeweiligen Koppelelements ferner eine Nase umfassen, die ausgebildet ist, um in eine zur Nase komplementäre Ausnehmung der jeweiligen Koppelaufnahme und/oder des Maskenkörpers einzugreifen, wenn der Hakenabschnitt mit der jeweiligen Koppelaufnahme verhakt wird. Die Nase kann eine spezielle Anschlagfläche aufweisen und ferner so ausgebildet sein, dass die Anschlagfläche der in die Ausnehmung eingreifenden Nase beim Bewegen des Koppelabschnitts relativ zur jeweiligen Koppelaufnahme in mindestens einer - vorzugsweise zu einer Wirkrichtung der von der Kopfbänderung ausgeübten Zugkraft parallelen - Bewegungsrichtung an einer speziellen Gegenfläche der Ausnehmung anschlägt, sodass ein weiteres Bewegen des Koppelabschnitts relativ zur jeweiligen Koppelaufnahme in der jeweiligen Bewegungsrichtung nicht möglich ist. Dabei kann ein Bewegen des Koppelabschnitts relativ zur jeweiligen Koppelaufnahme in mindestens einer von der Bewegungsrichtung abweichenden - vorzugsweise zur Bewegungsrichtung orthogonalen oder schrägen - weiteren Bewegungsrichtung in begrenztem Maß weiterhin möglich sein, ohne dass die Verhakung gelöst wird. Aufgrund eines derartigen begrenzten Spiels in der jeweiligen weiteren Bewegungsrichtung kann beispielsweise ein ungewolltes Lösen der Verhakung beim Verwenden der Atemmaske mit unterschiedlich breiten Gesichtern verhindert werden. Die Nase kann beispielsweise in einer Richtung hin zum Hakenabschnitt von einem übrigen Abschnitt des Koppelabschnitts oder des jeweiligen Koppelelements abstehen. Zusätzlich oder alternativ kann der Hakenabschnitt die Nase zumindest teilweise überragen. Es ist möglich, dass die Nase und der Hakenabschnitt von der gleichen Seite des jeweiligen Koppelelements abstehen. Die Nase kann beispielsweise im vorgenannten zweiten Längsabschnitt zusammen mit dem Hakenabschnitt angeordnet sein.

Gemäß einer Ausführungsform kann die Nase zumindest abschnittsweise keilförmig ausgebildet sein, um zu ermöglichen, dass die Nase beim kontrollierten Lösen der Verhakung allein aufgrund der von der Kopfbänderung ausgeübten Zugkraft aus der Ausnehmung gleitet. Dies kann den Bedienkomfort weiter verbessern.

Gemäß einer Ausführungsform kann der Hakenabschnitt eine verrundete Hakeninnenkontur aufweisen, die eine zur Hakeninnenkontur komplementäre verrundete Koppel(außen)kontur der jeweiligen Koppelaufnahme umgreifen kann, wenn der Hakenabschnitt mit der jeweiligen Koppelaufnahme verhakt ist. In diesem Fall kann die Verhakung so ausgebildet sein, dass die die Koppelkontur umgreifende Hakeninnenkontur - wenn der Maskenkörper mittels der Kopfbänderung gegenüber dem Gesicht des Patienten fixiert ist - durch die von der Kopfbänderung ausgeübte Zugkraft gegen die Koppelkontur gedrückt wird, um das ungewollte Lösen der Verhakung zu verhindern. Es ist möglich, dass die die Koppelkontur umgreifende Hakeninnenkontur beim Schwenken des Betätigungsabschnitts in der Schwenkrichtung zumindest teilweise relativ zur Koppelkontur bewegt wird, beispielsweise zumindest teilweise entlang der Koppelkontur gleitet, um eine Schwenkbewegung des Hakenabschnitts aus der jeweiligen Koppelaufnahme heraus zu ermöglichen. Eine derart geführte Schwenkbewegung kann das kontrollierte Lösen der Verhakung weiter vereinfachen.

Gemäß einer Ausführungsform kann ein freies Ende des Hakenabschnitts beim Bilden der Verhakung zusätzlich mit und/oder in der jeweiligen Koppelaufnahme verrastbar sein, sodass zum kontrollierten Lösen der Verhakung oder zum Schwenken des Betätigungsabschnitts in der Schwenkrichtung eine definierte Mindestkraft erforderlich ist. Die Mindestkraft zum Lösen der Verrastung, beispielsweise durch entsprechendes Drücken des Betätigungsabschnitts in der Schwenkrichtung, kann deutlich größer sein als eine Mindestkraft zum bloßen Schwenken des Betätigungsabschnitts in der Schwenkrichtung ohne eine derartige zusätzliche Verrastung. Beispielsweise kann das freie Ende, etwa in Form einer Spitze des Hakenabschnitts, beim Bilden der Verhakung zusätzlich in eine entsprechend geformte Vertiefung in der jeweiligen Koppelaufnahme form- und/oder kraftschlüssig einrasten, um das freie Ende mit der jeweiligen Koppelaufnahme zu verrasten.

Gemäß einer Ausführungsform kann der Koppelabschnitt von mindestens einem (oder jedem) der beiden Koppelelemente im gekoppelten Zustand (d. h., wenn der Koppelabschnitt mit der jeweiligen Koppelaufnahme gekoppelt ist) starr mit der jeweiligen Koppelaufnahme verbunden sein. Anders ausgedrückt ist im gekoppelten Zustand keine (maßgebliche) Bewegung des jeweiligen Koppelelements relativ zum Maskenkörper möglich. Im Gegensatz zu einer gelenkigen Kopplung hat dies die Wirkung, dass der Maskenkörper über das jeweilige Koppelelement mit einem bestimmten Drehmoment beaufschlagt werden kann. Somit kann beispielsweise durch Aufbringen eines entsprechenden Drehmoments auch bei einem größeren Maskenkörper einer Full-Face-Maske eine gleichmäßige Verteilung des Anpressdrucks über die Nasen- und Mundpartie gewährleistet werden, ohne dass dazu eine möglicherweise störende Stirnstütze oder zusätzliche Bänder erforderlich sind. Beispielsweise kann die Verhakung mit einer geeigneten Seitenführung ausgebildet sein, um zu verhindern, dass sich der Maskenkörper beim Tragen der Atemmaske relativ zum Koppelabschnitt ungewollt verdreht. Dadurch kann beispielsweise vermieden werden, dass der Maskenkörper - etwa aufgrund einer heftigen Bewegung des Kopfes - vom Gesicht wegkippt.

Gemäß einer Ausführungsform kann der Koppelabschnitt von mindestens einem (oder jedem) der beiden Koppelelemente eine keilförmige Außenkontur zum Einführen in die jeweilige Koppelaufnahme umfassen. In diesem Fall kann die jeweilige Koppelaufnahme eine zur Außenkontur komplementäre trichterförmige Innenkontur zum Aufnehmen der Außenkontur umfassen. Dies ermöglicht eine Selbstzentrierung des Koppelabschnitts gegenüber der Koppelaufnahme beim Einführen. Somit kann der Bedienkomfort weiter verbessert werden.

Gemäß einer Ausführungsform kann der Koppelabschnitt von mindestens einem (oder jedem) der beiden Koppelelemente über einen Biegeabschnitt mit einem übrigen Längsabschnitt des jeweiligen Koppelelements verbunden sein. Der Biegeabschnitt kann im Vergleich zum Koppelabschnitt und zum übrigen Längsabschnitt besonders biegsam ausgebildet sein, um ein Abwinkeln des Koppelabschnitts relativ zum übrigen Längsabschnitt zu ermöglichen. Dies ermöglicht die Verwendung der Atemmaske - insbesondere einer Variante der Atemmaske mit einer Verhakung, wie sie vor- und nachstehend beschrieben wird - mit (deutlich) verschieden breiten Gesichtern, ohne dass die Atemmaske an die jeweilige Gesichtsbreite eigens angepasst zu werden braucht. Wie weiter oben erwähnt, kann der Biegeabschnitt beispielsweise ein zwischen dem zweiten Längsabschnitt und dem dritten Längsabschnitt angeordneter vierter Längsabschnitt des jeweiligen Koppelelements sein.

Gemäß einer Ausführungsform kann eine Dicke des Biegeabschnitts in Längsrichtung des jeweiligen Koppelelements betrachtet entsprechend einem vorgegebenen - vorzugsweise periodischen oder sinusähnlichen - Dickenverlauf zwischen mindestens zwei verschiedenen Dickenwerten variieren. Beispielsweise kann die Dicke des Biegeabschnitts in Längsrichtung des jeweiligen Koppelelements betrachtet wellenförmig variieren. Anders ausgedrückt kann der Biegeabschnitt beispielsweise als eine Dünnstelle im Vergleich zum Koppelabschnitt und zum übrigen Längsabschnitt ausgebildet sein. Zusätzlich oder alternativ kann der Biegeabschnitt zumindest teilweise aus einem anderen Material als der Koppelabschnitt und/oder der übrige Längsabschnitt und/oder zumindest teilweise aus einem besonders weichen Material wie beispielsweise Silikon oder einem (vorzugsweise thermoplastischen) Elastomer sein. Alternativ kann der Biegeabschnitt als ein mechanisches Gelenk, d. h. als ein Gelenk mit mindestens zwei in mindestens einer Richtung bewegbar miteinander gekoppelten Gelenkteilen (beispielsweise in Form eines Scharniers oder eines Kugelgelenks), ausgebildet sein. Dies ermöglicht eine besonders flexible und dennoch stabile mechanische Verbindung zwischen dem Koppelabschnitt und dem übrigen Längsabschnitt. Ein solcher Biegeabschnitt kann zudem besonders einfach in großen Stückzahlen hergestellt werden, beispielsweise - je nach Ausführung - zusammen mit dem jeweiligen übrigen Koppelelement in einem Spritzgießverfahren.

Gemäß einer Ausführungsform kann der Koppelabschnitt von mindestens einem (oder jedem) der beiden Koppelelemente, vorzugsweise der Betätigungsabschnitt und/oder der Hakenabschnitt, eine Reißleinenaufnahme zum Befestigen einer Reißleine am Koppelabschnitt umfassen. Mithilfe der an der Reißleinenaufnahme befestigten Reißleine kann die Verhakung im Notfall durch entsprechendes Ziehen an der Reißleine, beispielsweise in einer Zugrichtung, die parallel zur Wirkrichtung der von der Kopfbänderung ausgeübten Zugkraft verläuft, schnell und sicher gelöst werden, ohne dass dazu eine Hand an den Betätigungsabschnitt geführt zu werden braucht.

### Kurze Beschreibung der Figuren

Im Folgenden werden beispielhafte Ausführungsformen mit Bezug auf die beigefügten Figuren beschrieben. Weder die Beschreibung noch die Figuren sind als Beschränkung des Umfangs der Offenbarung zu verstehen.
Fig. 1 zeigt eine perspektivische Ansicht einer beispielhaften Ausführungsform einer Atemmaske.
Fig. 2 zeigt eine Seitenansicht einer beispielhaften Ausführungsform einer Atemmaske.
Fig. 3 zeigt eine Seitenansicht eines Kopfes eines Patienten, der die Atemmaske aus Fig. 2 trägt.
Fig. 4 zeigt eine vergrößerte Ansicht eines Koppelelements einer beispielhaften Ausführungsform einer Atemmaske.
Fig. 5 zeigt eine Detailansicht einer Koppelaufnahme und eines Koppelabschnitts einer beispielhaften Ausführungsform einer Atemmaske im entkoppelten Zustand.
Fig. 6 zeigt eine Schnittansicht der Koppelaufnahme und des Koppelabschnitts aus Fig. 5 im gekoppelten Zustand.
Fig. 7 zeigt die Atemmaske aus Fig. 2 von vorn.
Fig. 8 zeigt die Atemmaske aus Fig. 2 von hinten.
Fig. 9 zeigt die Atemmaske aus Fig. 2 von oben.
Fig. 10 zeigt die Atemmaske aus Fig. 2 von unten.
Fig. 11 zeigt einen Maskenkörper und Koppelelemente einer beispielhaften Ausführungsform einer Atemmaske, bei der die Koppelelemente in den Maskenkörper eingehakt sind.
Fig. 12 zeigt eine Draufsicht auf den Maskenkörper und die Koppelelemente aus Fig. 11.
Fig. 13 zeigt einen Querschnitt durch den Maskenkörper und die Koppelelemente aus Fig. 11 und Fig. 12.
Fig. 14 zeigt eine vergrößerte Ansicht eines in Fig. 13 mit "A" markierten Teilbereichs.
Fig. 15 zeigt eine perspektivische Ansicht eines der Koppelelemente aus Fig. 11 bis Fig. 13.
Fig. 16 zeigt eine perspektivische Ansicht des Maskenkörpers aus Fig. 11 bis Fig. 13.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen

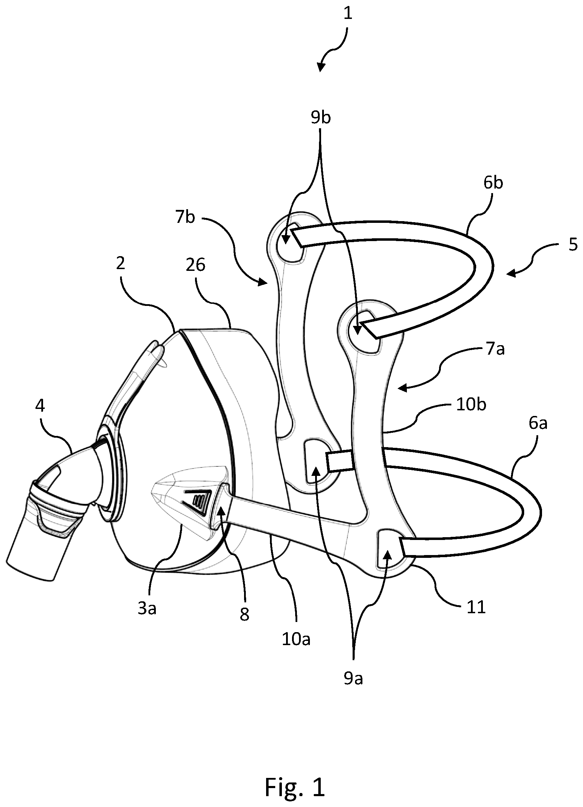

Fig. 1 zeigt eine perspektivische Ansicht einer Atemmaske 1 zum Versorgen eines Patienten mit Atemgas aus einer Atemgasquelle.

Die Atemmaske 1 umfasst einen Maskenkörper 2 mit einer ersten Koppelaufnahme 3a, einer zweiten Koppelaufnahme 3b und einem Schlauchanschluss 4 zum Anschließen eines mit der Atemgasquelle verbundenen Beatmungsschlauchs. In diesem Beispiel ist die Atemmaske 1 als Full-Face-Maske ausgeführt. Dementsprechend umschließt der Maskenkörper 2 bei Verwendung der Atemmaske 1 den Mund und die Nase des Patienten. Der Maskenkörper 2 kann zu diesem Zweck entsprechend gewölbt sein, sodass er einen Hohlkörper ausgebildet, in dem der Mund und die Nase platziert werden können.

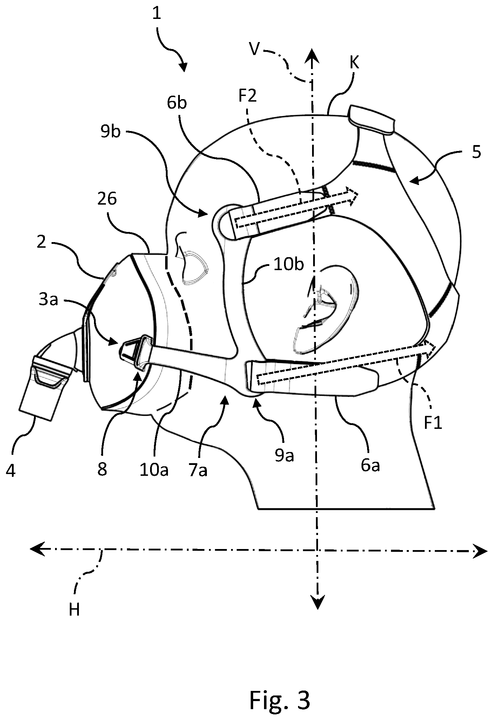

Des Weiteren umfasst die Atemmaske 1 eine Kopfbänderung 5 zum Fixieren des Maskenkörpers 2 gegenüber dem Gesicht des Patienten. Die Kopfbänderung 5 umfasst in diesem Beispiel ein erstes Band 6a und ein zweites Band 6b zum Spannen um den Kopf K des Patienten (siehe auch Fig. 3).

Darüber hinaus umfasst die Atemmaske 1 ein erstes Koppelelement 7a zum Koppeln der Kopfbänderung 5 mit der ersten Koppelaufnahme 3a und ein zweites Koppelelement 7b zum Koppeln der Kopfbänderung 5 mit der zweiten Koppelaufnahme 3b.

Jedes Koppelelement 7a, 7b umfasst einen Koppelabschnitt 8 zum Koppeln des Koppelelements 7a, 7b mit der jeweiligen Koppelaufnahme 3a bzw. 3b, eine erste Bandaufnahme 9a zum Befestigen des ersten Bands 6a und eine zweite Bandaufnahme 9b zum Befestigen des zweiten Bands 6b. Beispielsweise kann der Koppelabschnitt 8 im gekoppelten Zustand starr mit der jeweiligen Koppelaufnahme 3a bzw. 3b verbunden sein.

Wie aus Fig. 3 ersichtlich, ist jedes Koppelelement 7a, 7b so ausgebildet, dass - wenn der Maskenkörper 2 mittels der Kopfbänderung 5 gegenüber dem Gesicht des Patienten fixiert ist - die erste Bandaufnahme 9a in Richtung einer Sagittalachse H des Patienten betrachtet (hier in Richtung einer Horizontalen H betrachtet) zumindest teilweise zwischen dem Kinn und einem Ohr des Patienten und in Richtung einer zur Sagittalachse H orthogonalen Longitudinalachse V des Patienten betrachtet (hier in Richtung einer Vertikalen V betrachtet) zumindest teilweise zwischen dem Kinn und der Schläfenregion des Patienten angeordnet ist. Dabei ist die zweite Bandaufnahme 9b zumindest teilweise gegenüber der Schläfenregion des Patienten angeordnet.

In diesem Beispiel ist die erste Bandaufnahme 9a zumindest teilweise gegenüber einem Teil des Unterkiefers des Patienten, insbesondere gegenüber einem Unterkieferast, angeordnet. Zudem kann der mit der jeweiligen Koppelaufnahme 3a bzw. 3b gekoppelte Koppelabschnitt 8 in Richtung der Longitudinalachse V betrachtet zumindest teilweise zwischen der ersten Bandaufnahme 9a und der zweiten Bandaufnahme 9b angeordnet sein. Dabei kann die zweite Bandaufnahme 9b in Richtung der Sagittalachse H betrachtet zumindest teilweise zwischen dem Koppelabschnitt 8 und der ersten Bandaufnahme 9a angeordnet sein. Anders ausgedrückt kann die zweite Bandaufnahme 9b in Richtung der Sagittalachse H betrachtet deutlich näher am Maskenkörper 2 als die erste Bandaufnahme 9a angeordnet sein.

Bei Verwendung der Atemmaske 1 übt das erste Band 6a eine erste Zugkraft F1 auf die erste Bandaufnahme 9a eines jeden Koppelelements 7a, 7b aus. In entsprechender Weise übt das zweite Band 6b eine zweite Zugkraft F2 auf die zweite Bandaufnahme 9b eines jeden Koppelelements 7a, 7b aus.

Dabei kann die erste Zugkraft F1 zur Nackenregion und/oder zum Hinterhauptbein des Patienten gerichtet sein, wohingegen die zweite Zugkraft F2 zu einem Kopfabschnitt außerhalb der Nackenregion und/oder des Hinterhauptbeins des Patienten, insbesondere zum Scheitelbein des Patienten, gerichtet sein kann. Dementsprechend kann das erste Band 6a unterhalb der Ohren um die Nackenregion und/oder das Hinterhauptbein gespannt sein und/oder das zweite Band 6b oberhalb der Ohren um den außerhalb der Nackenregion und/oder des Hinterhauptbeins befindlichen Kopfabschnitt, insbesondere um das Scheitelbein, gespannt sein.

Es ist möglich, dass eine in Richtung der Sagittalachse H wirkende Komponente der ersten Zugkraft F1 deutlich größer als, vorzugsweise mindestens doppelt so groß wie eine in Richtung der Longitudinalachse V wirkende Komponente der ersten Zugkraft F1 ist. Dies kann in entsprechender Weise auch für die zweite Zugkraft F2 zutreffen.

Zudem können die erste Zugkraft F1 und die zweite Zugkraft F2 - wie hier angedeutet - (annähernd) parallel zueinander ausgerichtet sein.

Aufgrund der beiden Zugkräfte F1, F2 wird der Maskenkörper 2 derart gegen das Gesicht gedrückt, dass eine luftdichte Verbindung zwischen dem Maskenkörper 2 und dem Gesicht hergestellt wird.

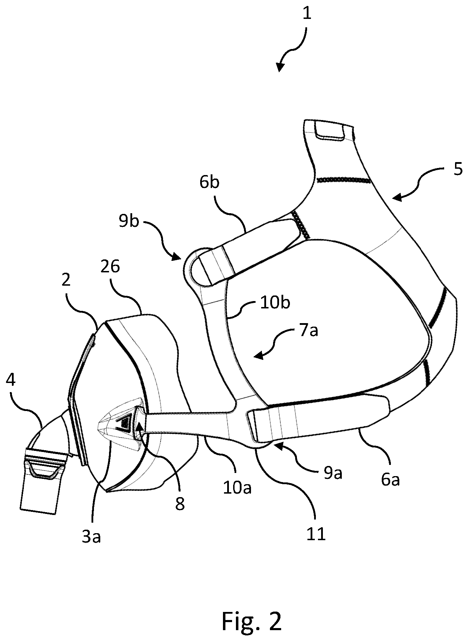

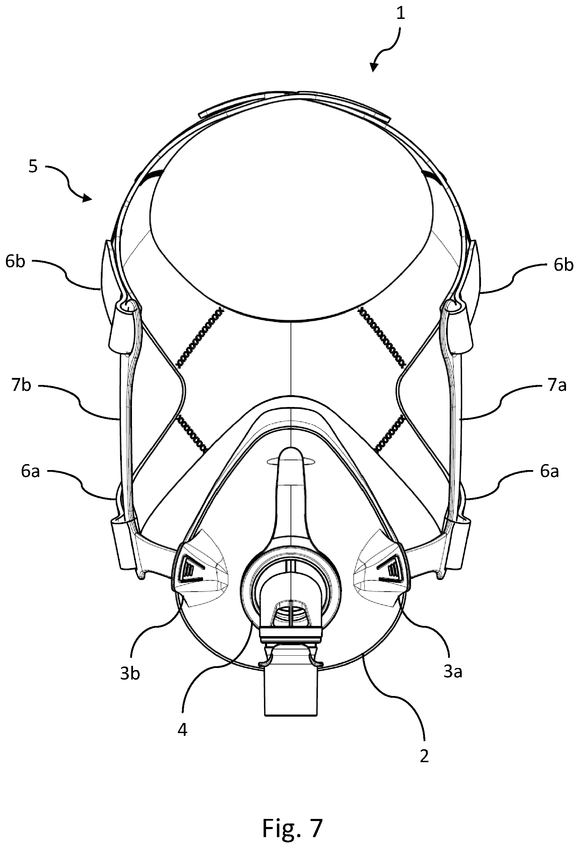

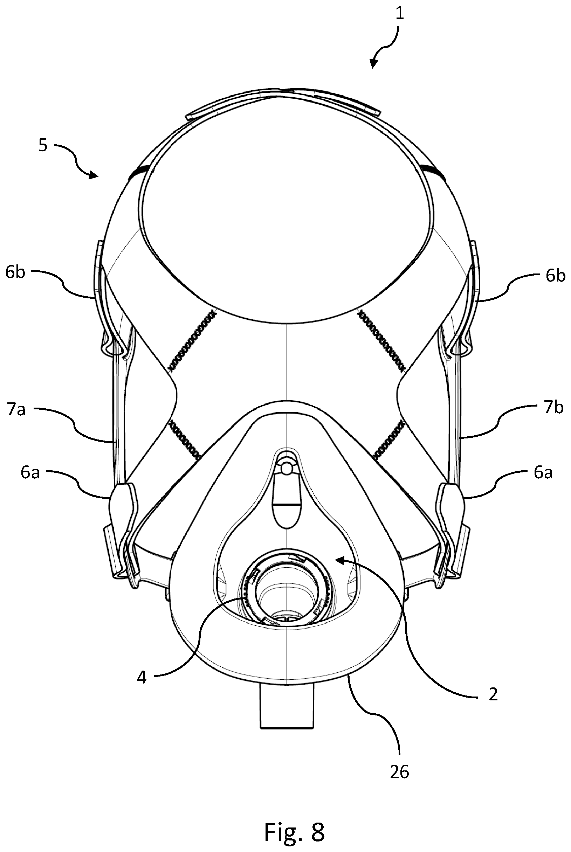

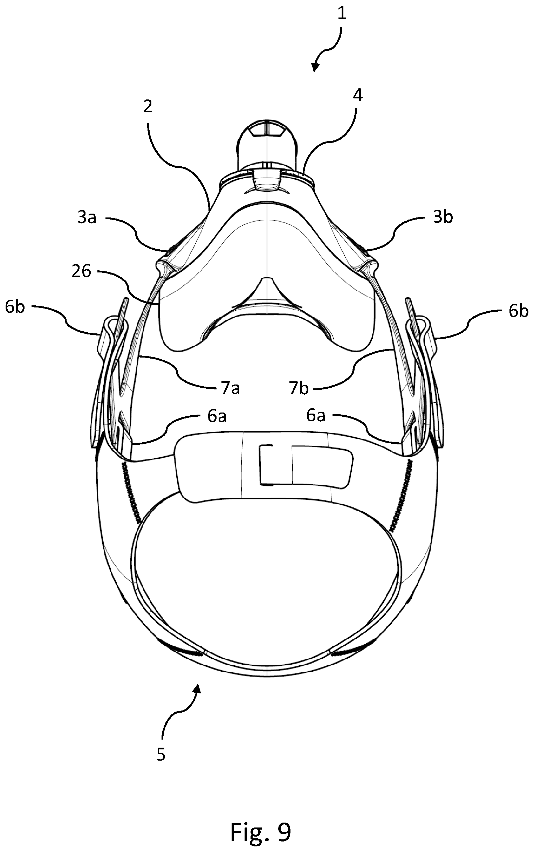

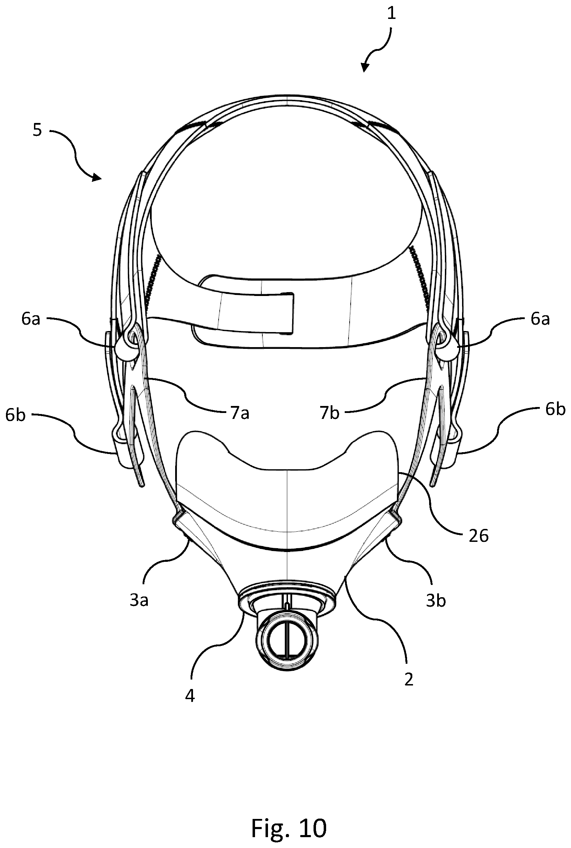

Das erste Band 6a und das zweite Band 6b können - wie in Fig. 1 gezeigt - voneinander getrennte Bänder sein oder - wie in Fig. 2, Fig. 3 und Fig. 7 bis Fig. 10 gezeigt - miteinander zu einem Bandverbund kombiniert sein.

Optional kann jedes Koppelelement 7a, 7b einen ersten Arm 10a und einen zweiten Arm 10b umfassen, die L- oder V-förmig miteinander verbunden sein können und in einem Übergangsabschnitt 11 ineinander übergehen können. Dabei kann - wie hier gezeigt - die erste Bandaufnahme 9a zumindest teilweise im Übergangsabschnitt 11 angeordnet sein, die zweite Bandaufnahme 9b an einem dem Übergangsabschnitt 11 abgewandten Ende des zweiten Arms 10b angeordnet sein und der Koppelabschnitt 8 an einem dem Übergangsabschnitt 11 abgewandten Ende des ersten Arms 10a angeordnet sein.

Zusätzlich können die beiden Bandaufnahmen 9a, 9b eines jeden Koppelelements 7a, 7b eine im Material des jeweiligen Koppelelements 7a bzw. 7b ausgebildete Aufnahmeöffnung 12 zum Einführen des jeweiligen Bands 6a bzw. 6b umfassen. Beispielsweise können die Endabschnitte der Bänder 6a, 6b durch die entsprechende Aufnahmeöffnung 12 hindurchgeführt und in geeigneter Weise an einem restlichen Abschnitt der Kopfbänderung 5, beispielsweise einem restlichen Abschnitt des jeweiligen Bands 6a bzw. 6b, befestigt werden, um eine Schlaufe zu bilden. Die Befestigung kann vorzugsweise über eine Klettverbindung erfolgen.

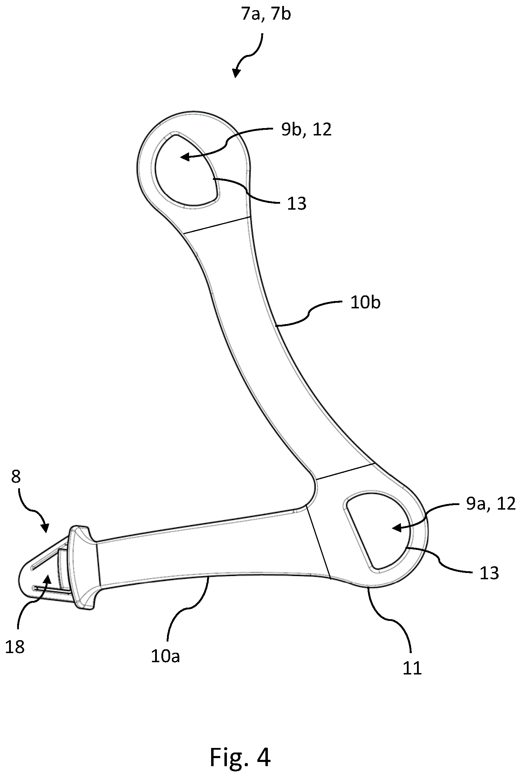

Wie in Fig. 4 zu erkennen, kann die Aufnahmeöffnung 12 zumindest teilweise durch einen in einer Umfangsrichtung der Aufnahmeöffnung 12 gekrümmten, beispielsweise kreisbogenförmigen Randabschnitt 13 begrenzt sein. Das an der jeweiligen Bandaufnahme 9a bzw. 9b befestigte, beispielsweise zu einer Schlaufe verbundene Band 6a bzw. 6b kann den Randabschnitt 13 zumindest teilweise umschlingen. Dementsprechend kann eine Ausrichtung einer Längsachse des jeweiligen Bands 6a bzw. 6b relativ zur Sagittalachse H durch Verschieben des den Randabschnitt 13 umschlingenden Teils des jeweiligen Bands 6a bzw. 6b entlang des Randabschnitts 13 veränderbar sein. Der Randabschnitt 13 kann deutlich länger sein, als der den Randabschnitt 13 umschlingende Teil des jeweiligen Bands 6a bzw. 6b breit ist. Somit wird eine ausreichende Verstellung in beide Richtungen ermöglicht.

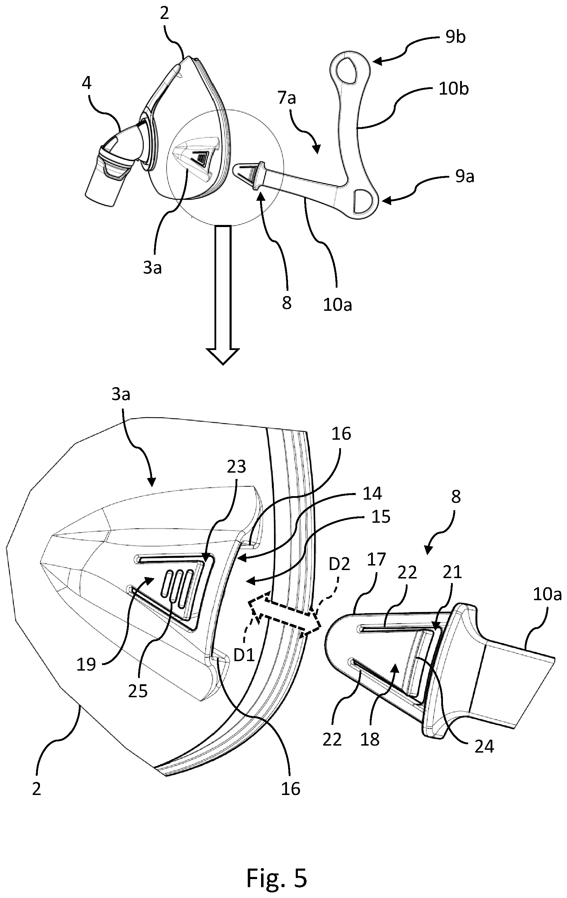

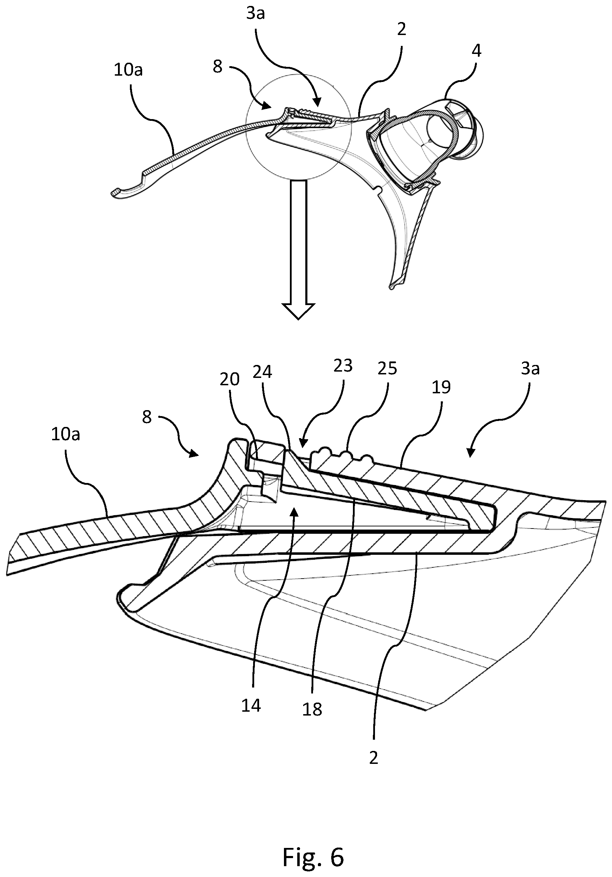

Wie in Fig. 5 und Fig. 6 am Beispiel der ersten Koppelaufnahme 3a gezeigt, kann jede Koppelaufnahme 3a, 3b einen Aufnahmeraum 14 mit einer Einführöffnung 15 zum Einführen des jeweiligen Koppelabschnitts 8 in einer Einführrichtung D1 umfassen.

Der Aufnahmeraum 14 kann beispielsweise eine trichterförmige Innenkontur 16 aufweisen. Dementsprechend kann der Koppelabschnitt 8 mit einer zur Innenkontur 16 komplementären keilförmigen Außenkontur 17 zum Einführen in die Innenkontur 16 ausgebildet sein. Dies ermöglicht eine Selbstzentrierung des Koppelabschnitts 8 gegenüber der Koppelaufnahme 3a bzw. 3b beim Einführen durch die Einführöffnung 15.

Zudem kann der Koppelabschnitt 8 mit der Koppelaufnahme 3a bzw. 3b zu einer Steckverbindung verbindbar sein.

Dazu kann ein Verriegelungselement 18 am Koppelabschnitt 8 zwischen einer Verriegelungsstellung und einer Entriegelungsstellung bewegbar gelagert sein. Das Verriegelungselement 18 kann ausgebildet sein, um die Steckverbindung in der Verriegelungsstellung zu verriegeln, sodass ein Verlagern des Koppelabschnitts 8 in einer der Einführrichtung D1 entgegengesetzten Trennrichtung D2 weg von der Koppelaufnahme 3a bzw. 3b nicht möglich ist, und in der Entriegelungsstellung zu entriegeln, sodass das Verlagern des Koppelabschnitts 8 in der Trennrichtung D2 (zumindest in begrenztem Maß) möglich ist.

Ferner kann ein Betätigungselement 19 an der Koppelaufnahme 3a bzw. 3b zwischen einer Ruhestellung und einer Betätigungsstellung bewegbar gelagert sein. Das Betätigungselement 19 dient zur Betätigung des Verriegelungselements 18, wenn die Steckverbindung verriegelt ist. Das Betätigungselement 19 kann ausgebildet sein, um beim Bewegen in die Betätigungsstellung gegen das Verriegelungselement 18 zu drücken, sodass das Verriegelungselement 18 in die Entriegelungsstellung bewegt wird.

Zusätzlich kann das Betätigungselement 19 ausgebildet sein, um einen Verlagerungsweg des Koppelabschnitts 8 beim Verlagern in der Trennrichtung D2 in der Betätigungsstellung zu begrenzen, sodass der Koppelabschnitt 8 nur bis in eine Zwischenstellung relativ zur Koppelaufnahme 3a bzw. 3b verlagert werden kann, und in der Ruhestellung nicht zu begrenzen, sodass der Koppelabschnitt 8 über die Zwischenstellung hinaus verlagert werden kann. Die Steckverbindung wird erst dann vollständig getrennt, wenn der Koppelabschnitt 8 über die Zwischenstellung hinaus verlagert wird.

Es ist möglich, dass das Verriegelungselement 18 durch Verlagern des Koppelabschnitts 8 bis in die Zwischenstellung gegenüber einer Anschlagkante 20 der Koppelaufnahme 3a bzw. 3b positionierbar ist. Die Anschlagkante 20 kann ausgebildet sein, um das gegenüber der Anschlagkante 20 positionierte, beispielsweise an der Anschlagkante 20 anliegende Verriegelungselement 18 daran zu hindern, in die Verriegelungsstellung zurückzukehren.

Beispielsweise kann das Betätigungselement 19 in der Betätigungsstellung in eine keilförmige Vertiefung 21 in dem in den Aufnahmeraum 14 eingeführten Koppelabschnitt 8 eingreifen. Dementsprechend kann das Betätigungselement 19 durch Zurückbewegen in die Ruhestellung aus der Vertiefung 21 herausbewegbar sein. Die Vertiefung 21 kann in einer Richtung quer zur Trennrichtung D2 durch zwei schräg zueinander ausgerichtete Begrenzungskanten 22 begrenzt sein. Die Begrenzungskanten 22 können derart zueinander ausgerichtet sein, dass sie in der Einführrichtung D1 betrachtet aufeinander zulaufen.

Die Steckverbindung kann so ausgebildet sein, dass ein in die Vertiefung 21 eingreifender Abschnitt des Betätigungselements 19 beim Verlagern des Koppelabschnitts 8 in der Trennrichtung D2 auf die Begrenzungskanten 22 zubewegt wird und - wenn der Koppelabschnitt 8 die Zwischenstellung erreicht - beidseitig an den Begrenzungskanten 22 anschlägt, sodass ein weiteres Verlagern des Koppelabschnitts 8 über die Zwischenstellung hinaus nicht möglich ist. Erst wenn das Betätigungselement 19 wieder aus der Vertiefung 21 herausbewegt wird, sodass der Verlagerungsweg freigegeben wird, kann die Steckverbindung vollständig getrennt werden.

Das Betätigungselement 19 kann beispielsweise als eine Betätigungslasche 19 ausgebildet sein. Die Betätigungslasche 19 kann an einem ihrer Enden mit der Koppelaufnahme 3a bzw. 3b - genauer mit einem Rand, der eine Durchgangsöffnung 23 in einem Wandabschnitt des Aufnahmeraums 14 begrenzt - verbunden sein und durch elastisches Verbiegen ihres anderen, unverbundenen Endes in die Betätigungsstellung bewegbar sein, sodass auf die Betätigungslasche 19 in der Betätigungsstellung eine Federkraft zum Zurückstellen der Betätigungslasche 19 in die Ruhestellung einwirkt. Beim Bewegen in die Betätigungsstellung kann die Betätigungslasche 19 beispielsweise durch die Durchgangsöffnung 23 hindurch ins Innere des Aufnahmeraums 14 geschwenkt werden.

Zusätzlich oder alternativ kann das Verriegelungselement 18 als eine Verriegelungslasche 18 ausgebildet sein. Die Verriegelungslasche 18 kann an einem ihrer Enden mit dem Koppelabschnitt 8 - genauer mit einem die Vertiefung 21 begrenzenden Rand des Koppelabschnitts 8 - verbunden sein und durch elastisches Verbiegen ihres anderen, unverbundenen Endes in die Entriegelungsstellung bewegbar sein, sodass auf die Verriegelungslasche 18 in der Entriegelungsstellung eine Federkraft zum Zurückstellen der Verriegelungslasche 18 in die Verriegelungsstellung einwirkt. Beispielsweise kann die Verriegelungslasche 18 beim Bewegen in die Entriegelungsstellung zusammen mit der gegen die Verriegelungslasche 18 drückenden Betätigungslasche 19 in die Vertiefung 21 hineingeschwenkt werden.

Die Vertiefung 21 kann sich zumindest teilweise innerhalb eines von der Außenkontur 17 umgebenen Teilabschnitts des Koppelabschnitts 8 erstrecken.

Es ist möglich, dass die Verriegelungslasche 18 an ihrem unverbundenen Ende einen Vorsprung 24 aufweist, der in der Verriegelungsstellung formschlüssig in die Koppelaufnahme 3a bzw. 3b, beispielsweise in die Durchgangsöffnung 23, eingreift und in der Entriegelungsstellung die Koppelaufnahme 3a bzw. 3b freigibt.

Die Betätigungslasche 19 kann zusätzlich eine speziell strukturiere, beispielsweise besonders ergonomische und/oder besonders rutschfeste Berührungsfläche 25 aufweisen, an der die Betätigungslasche 19 mit einem Finger gedrückt werden kann, um die Steckverbindung zu entriegeln. Die Berührungsfläche 25 kann beispielsweise so strukturiert, vorzugsweise geriffelt, sein, dass ein Verrutschen eines die Berührungsfläche 25 drückenden Fingers in einer Richtung parallel zur Einführrichtung D1 bzw. zur Trennrichtung D2 erschwert wird.

Zusätzlich kann die Atemmaske 1 einen Maskenwulst 26 zum Abstützen des Maskenkörpers 2 an der Gesichtshaut des Patienten umfassen. Somit kann der Maskenkörper 2 über den Maskenwulst 26 an das Gesicht angelegt werden. Der Maskenwulst 26 kann deutlich flexibler als der Maskenkörper 2 ausgebildet sein und den Maskenkörper 2 bei Verwendung der Atemmaske 1 luftdicht mit der Gesichtshaut verbinden.

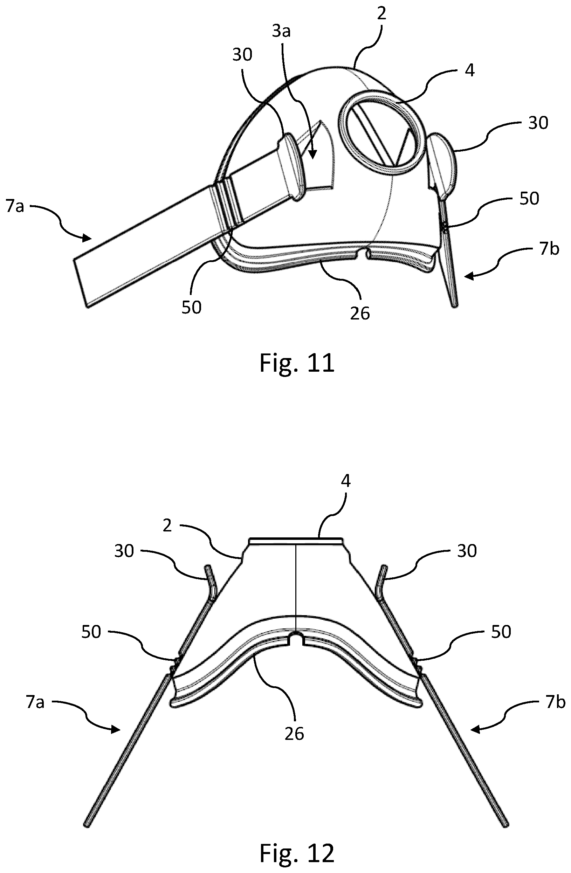

Fig. 11 zeigt eine Variante, bei der der Maskenkörper 2 mit den beiden Koppelelementen 7a, 7b über eine Verhakung statt über eine Steckverbindung wiederlösbar verbunden werden kann.

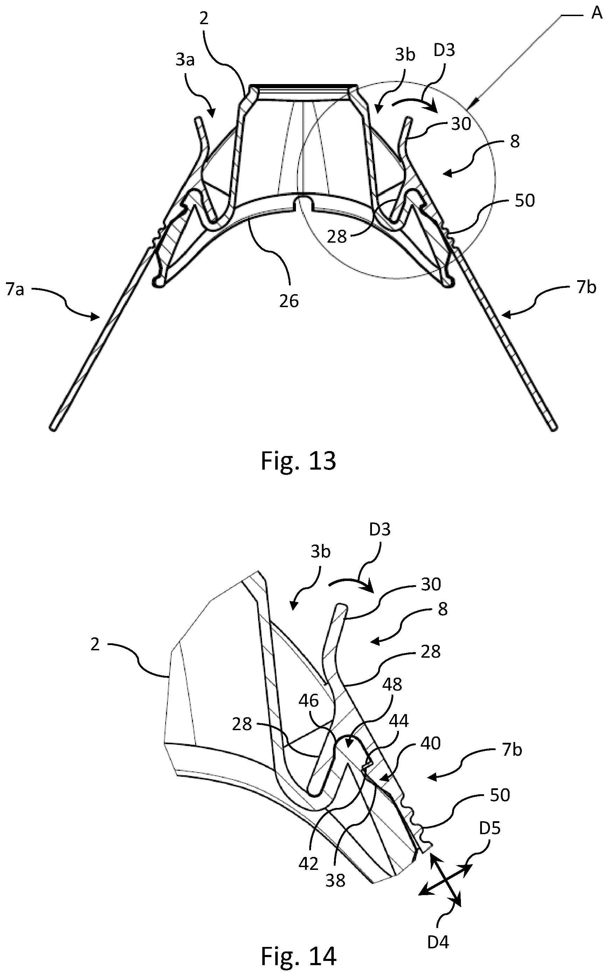

Wie in Fig. 13 bis Fig. 15 zu sehen, umfasst in diesem Beispiel der Koppelabschnitt 8 eines jeden Koppelelements 7a, 7b einen hakenförmigen Hakenabschnitt 28, der mit der jeweiligen Koppelaufnahme 3a bzw. 3b verhakbar ist, um eine Verhakung zum Befestigen der Kopfbänderung 5 am Maskenkörper 2 zu bilden. Des Weiteren umfasst der Koppelabschnitt 8 eines jeden Koppelelements 7a, 7b einen flügelförmigen Betätigungsabschnitt 30, der mit dem Hakenabschnitt 28 fest verbunden ist und - wenn der Maskenkörper 2 mittels der Kopfbänderung 5 gegenüber dem Gesicht des Patienten fixiert ist - derart vom übrigen Koppelabschnitt 8 absteht, dass er mit mindestens einem Finger in einer Schwenkrichtung D3 weg von der jeweiligen Koppelaufnahme 3a bzw. 3b, also jeweils weg vom Maskenkörper 2, geschwenkt werden kann.

Die Verhakung ist zudem so ausgebildet, dass der Hakenabschnitt 28 - wenn der Maskenkörper 2 mittels der Kopfbänderung 5 gegenüber dem Gesicht des Patienten fixiert ist - durch eine von der Kopfbänderung 5 ausgeübte Zugkraft gegen die jeweilige Koppelaufnahme 3a bzw. 3b gedrückt wird. Dadurch kann ein ungewolltes, beispielweise versehentliches Lösen der Verhakung verhindert werden. Ferner ist die Verhakung so ausgebildet, dass der Hakenabschnitt 28 durch Schwenken des Betätigungsabschnitts 30 in der Schwenkrichtung D3 aus der jeweiligen Koppelaufnahme 3a bzw. 3b herausbewegbar ist. Dies ermöglicht ein kontrolliertes Lösen der Verhakung.

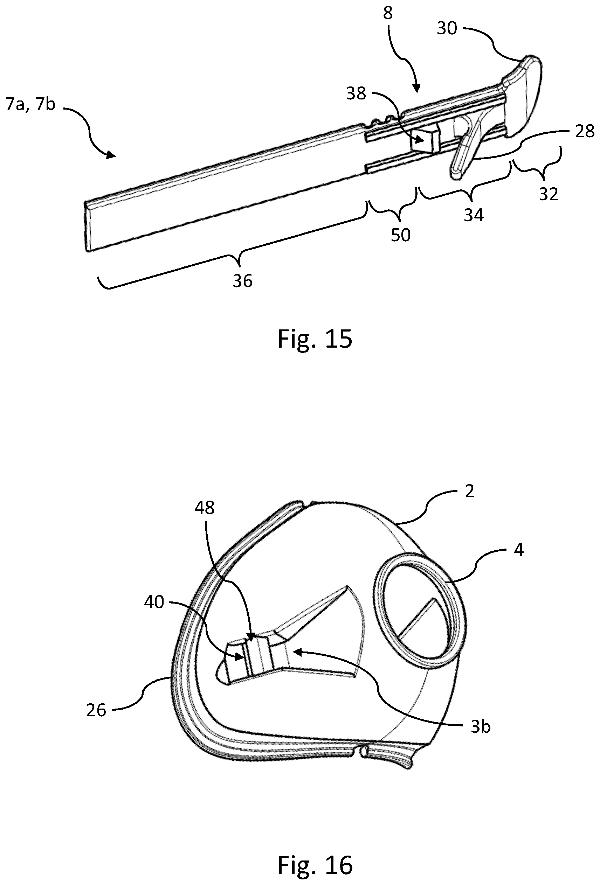

Wie in Fig. 15 gut zu erkennen, kann das Koppelelement 7a bzw. 7b in dessen Längsrichtung betrachtet in mindestens einen ersten Längsabschnitt 32, einen zweiten Längsabschnitt 34 und einen dritten Längsabschnitt 36 unterteilt sein. Dabei kann der zweite Längsabschnitt 34 zwischen dem ersten Längsabschnitt 32 und dem dritten Längsabschnitt 36 angeordnet sein. Zudem kann der erste Längsabschnitt 32 schräg oder gerade von einem (hier dem dritten Längsabschnitt 36 abgewandten) Ende des zweiten Längsabschnitts 34 abstehen. Dieser abstehende Teil des ersten Längsabschnitts 32 kann als der Betätigungsabschnitt 30 fungieren. Des Weiteren kann der zweite Längsabschnitt 34 den Hakenabschnitt 28 umfassen. Bei dem dritten Längsabschnitt 36 kann es sich um einen (mehr oder weniger langen) übrigen Längsabschnitt des jeweiligen Koppelelements 7a bzw. 7b handeln, der an seinem dem zweiten Längsabschnitt 34 abgewandten Ende mit dem jeweiligen Band der Kopfbänderung 5 in geeigneter Weise verbunden sein kann.

Zusätzlich kann der Koppelabschnitt 8 des jeweiligen Koppelelements 7a bzw. 7b, beispielsweise dessen zweiter Längsabschnitt 34, eine Nase 38 umfassen, die ausgebildet sein kann, um in eine zur Nase 38 komplementäre Ausnehmung 40 der jeweiligen Koppelaufnahme 3a bzw. 3b und/oder des Maskenkörpers 2 einzugreifen, wenn der Hakenabschnitt 28 mit der jeweiligen Koppelaufnahme 3a bzw. 3b verhakt wird. Der Hakenabschnitt 28 kann beispielsweise - wie in Fig. 15 gezeigt - in Längsrichtung des jeweiligen Koppelelements 7a bzw. 7b betrachtet zumindest teilweise zwischen der Nase 38 und dem Betätigungsabschnitt 30 angeordnet sein. Zudem können die Nase 38 und der Hakenabschnitt 28 jeweils von der gleichen Seite des jeweiligen Koppelelements 7a bzw. 7b abstehen, wobei der Hakenabschnitt 28 die Nase 38 teilweise oder vollständig überragen kann.

Wie in Fig. 14 zu sehen, kann die Nase 38 eine Anschlagfläche 42 aufweisen und ferner so ausgebildet sein, dass die Anschlagfläche 42 der in die Ausnehmung 40 eingreifenden Nase 38 beim Bewegen des Koppelabschnitts 8 relativ zur jeweiligen Koppelaufnahme 3a bzw. 3b in mindestens einer - hier zu einer Wirkrichtung der von der Kopfbänderung 5 ausgeübten Zugkraft parallelen - Bewegungsrichtung D4 an einer Gegenfläche 44 der Ausnehmung 40 anschlägt, sodass ein weiteres Bewegen des Koppelabschnitts 8 relativ zur jeweiligen Koppelaufnahme 3a bzw. 3b in der jeweiligen Bewegungsrichtung D4 nicht möglich ist. Dennoch kann dabei ein Bewegen des Koppelabschnitts 8 relativ zur jeweiligen Koppelaufnahme 3a bzw. 3b in mindestens einer von der Bewegungsrichtung D4 abweichenden - hier zur Bewegungsrichtung D4 orthogonalen - weiteren Bewegungsrichtung D5 (genauer eine Relativbewegung zwischen der Anschlagfläche 42 und der die Anschlagfläche 42 berührenden Gegenfläche 44 in der weiteren Bewegungsrichtung D5) in begrenztem Maß weiterhin möglich sein, ohne dass sich die Verhakung löst.

Zweckmäßigerweise kann die Nase 38 zumindest abschnittsweise keilförmig ausgebildet sein. Dies ermöglicht es, dass die Nase 38 beim kontrollierten Lösen der Verhakung allein aufgrund der von der Kopfbänderung 5 ausgeübten Zugkraft aus der Ausnehmung 40 gleitet.

Zudem kann der Hakenabschnitt 28 eine verrundete Hakeninnenkontur 46 aufweisen, die eine zur Hakeninnenkontur 46 komplementäre verrundete Koppelkontur 48 der jeweiligen Koppelaufnahme 3a bzw. 3b umgreift, wenn der Hakenabschnitt 28 mit der jeweiligen Koppelaufnahme 3a bzw. 3b verhakt ist. Die Verhakung kann so ausgebildet sein, dass die die Koppelkontur 48 umgreifende Hakeninnenkontur 46 - wenn der Maskenkörper 2 mittels der Kopfbänderung 5 gegenüber dem Gesicht des Patienten fixiert ist - durch die von der Kopfbänderung 5 ausgeübte Zugkraft gegen die Koppelkontur 48 gedrückt wird, um das ungewollte Lösen der Verhakung zu verhindern, und beim Schwenken des Betätigungsabschnitts 30 in der Schwenkrichtung D3 zumindest teilweise entlang der Koppelkontur 48 gleitet, um eine entsprechende Schwenkbewegung des Hakenabschnitts 28 aus der jeweiligen Koppelaufnahme 3a bzw. 3b heraus und somit das kontrollierte Lösen der Verhakung zu ermöglichen.

Darüber hinaus kann der Koppelabschnitt 8 des jeweiligen Koppelelements 7a bzw. 7b, beispielsweise der zweite Längsabschnitt 34, über einen speziellen Biegeabschnitt 50 mit einem übrigen Längsabschnitt des jeweiligen Koppelelements 7a bzw. 7b, beispielsweise dem dritten Längsabschnitt 36, verbunden sein. Der Biegeabschnitt 50 kann im Vergleich zum Koppelabschnitt 8 und zum übrigen Längsabschnitt besonders biegsam ausgebildet sein. Dadurch wird ein Abwinkeln des Koppelabschnitts 8 relativ zum übrigen Längsabschnitt ermöglicht, was beispielweise bei der Verwendung der Atemmaske 1 mit unterschiedlich breiten Gesichtern eine Rolle spielt. Beispielsweise kann eine Dicke des Biegeabschnitts 50 in Längsrichtung des jeweiligen Koppelelements 7a bzw. 7b betrachtet entsprechend einem vorgegebenen - hier wellenartigen oder sinusähnlichen - Dickenverlauf zwischen mindestens zwei verschiedenen Dickenwerten variieren. Dies ermöglicht eine besonders flexible und dennoch stabile mechanische Verbindung zwischen dem Koppelabschnitt 8 und dem übrigen Längsabschnitt. Dabei kann der Biegeabschnitt 50 aus dem gleichen Material (beispielsweise dem gleichen, vorzugsweise thermoplastischen Kunststoffmaterial) wie das übrige Koppelelement 7a bzw. 7b ausgebildet sein.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Bezugszeichenliste

- 1: Atemmaske
- 2: Maskenkörper
- 3a: erste Koppelaufnahme
- 3b: zweite Koppelaufnahme
- 4: Schlauchanschluss
- 5: Kopfbänderung
- 6a: erstes Band
- 6b: zweites Band
- 7a: erstes Koppelelement
- 7b: zweites Koppelelement
- 8: Koppelabschnitt
- 9a: erste Bandaufnahme
- 9b: zweite Bandaufnahme
- 10a: erster Arm
- 10b: zweiter Arm
- 11: Übergangsabschnitt
- 12: Aufnahmeöffnung
- 13: Randabschnitt
- 14: Aufnahmeraum
- 15: Einführöffnung
- 16: Innenkontur
- 17: Außenkontur
- 18: Verriegelungselement, Verriegelungslasche
- 19: Betätigungselement, Betätigungslasche
- 20: Anschlagkante
- 21: Vertiefung
- 22: Begrenzungskante
- 23: Durchgangsöffnung
- 24: Vorsprung
- 25: Berührungsfläche
- 26: Maskenwulst
- 28: Hakenabschnitt
- 30: Betätigungsabschnitt
- 32: erster Längsabschnitt
- 34: zweiter Längsabschnitt
- 36: dritter Längsabschnitt
- 38: Nase
- 40: Ausnehmung
- 42: Anschlagfläche
- 44: Gegenfläche
- 46: Hakeninnenkontur
- 48: Koppelkontur
- 50: Biegeabschnitt
- D1: Einführrichtung
- D2: Trennrichtung
- D3: Schwenkrichtung
- D4: Bewegungsrichtung
- D5: weitere Bewegungsrichtung
- F1: erste Zugkraft
- F2: zweite Zugkraft
- H: Sagittalachse, Horizontale
- K: Kopf
- V: Longitudinalachse, Vertikale

## Patentansprüche

1. Atemmaske (1) zum Versorgen eines Patienten mit Atemgas, wobei die Atemmaske (1) umfasst:
einen Maskenkörper (2) zum Anlegen an den Mund und/oder die Nase des Patienten, wobei der Maskenkörper (2) eine erste Koppelaufnahme (3a), eine zweite Koppelaufnahme (3b) und einen Schlauchanschluss (4) zum Anschließen eines Beatmungsschlauchs umfasst;
eine Kopfbänderung (5) zum Fixieren des Maskenkörpers (2) gegenüber dem Gesicht des Patienten;
ein erstes Koppelelement (7a) zum Koppeln der Kopfbänderung (5) mit der ersten Koppelaufnahme (3a);
ein zweites Koppelelement (7b) zum Koppeln der Kopfbänderung (5) mit der zweiten Koppelaufnahme (3b);
wobei jedes Koppelelement (7a, 7b) einen Koppelabschnitt (8) zum Koppeln des Koppelelements (7a, 7b) mit der jeweiligen Koppelaufnahme (3a, 3b) und eine Bandaufnahme (9a, 9b) zum Befestigen eines Bands (6a, 6b) der Kopfbänderung (5) umfasst, wobei der Koppelabschnitt (8) von mindestens einem der Koppelelemente (7a, 7b) mit der jeweiligen Koppelaufnahme (3a, 3b) zu einer Steckverbindung verbindbar ist;
wobei die Atemmaske (1) ferner umfasst:
ein am Maskenkörper (2) und/oder am jeweiligen Koppelelement (7a, 7b) zwischen einer Verriegelungsstellung und einer Entriegelungsstellung bewegbar gelagertes Verriegelungselement (18), das ausgebildet ist, um die Steckverbindung in der Verriegelungsstellung zu verriegeln, sodass ein Verlagern des Koppelabschnitts (8) in einer Trennrichtung (D2) weg von der jeweiligen Koppelaufnahme (3a, 3b) nicht möglich ist, und in der Entriegelungsstellung zu entriegeln, sodass das Verlagern des Koppelabschnitts (8) in der Trennrichtung (D2) möglich ist;
ein am Maskenkörper (2) und/oder am jeweiligen Koppelelement (7a, 7b) zwischen einer Ruhestellung und einer Betätigungsstellung bewegbar gelagertes Betätigungselement (19) zum Betätigen des Verriegelungselements (18), wenn die Steckverbindung verriegelt ist, wobei das Betätigungselement (19) ausgebildet ist, um beim Bewegen in die Betätigungsstellung gegen das Verriegelungselement (18) zu drücken, sodass das Verriegelungselement (18) in die Entriegelungsstellung bewegt wird.

2. Atemmaske (1) nach Anspruch 1,
wobei das Betätigungselement (19) ausgebildet ist, um einen Verlagerungsweg des Koppelabschnitts (8) beim Verlagern in der Trennrichtung (D2) in der Betätigungsstellung zu begrenzen, sodass der Koppelabschnitt (8) nur bis in eine Zwischenstellung relativ zur jeweiligen Koppelaufnahme (3a, 3b) verlagert werden kann, und in der Ruhestellung nicht zu begrenzen, sodass der Koppelabschnitt (8) über die Zwischenstellung hinaus verlagert werden kann, wobei die Steckverbindung durch Verlagern des Koppelabschnitts (8) über die Zwischenstellung hinaus trennbar ist.

3. Atemmaske (1) nach Anspruch 2,
wobei das Verriegelungselement (18) durch Verlagern des Koppelabschnitts (8) bis in die Zwischenstellung gegenüber einer Anschlagkante (20) des Maskenkörpers (2) und/oder des jeweiligen Koppelelements (7a, 7b) positionierbar ist, wobei die Anschlagkante (20) ausgebildet ist, um das gegenüber der Anschlagkante (20) positionierte Verriegelungselement (18) daran zu hindern, in die Verriegelungsstellung zurückzukehren.

4. Atemmaske (1) nach Anspruch 2 oder 3,
wobei das Betätigungselement (19) in der Betätigungsstellung in eine keilförmige Vertiefung (21) im Maskenkörper (2) und/oder im jeweiligen Koppelelement (7a, 7b) eingreift und durch Zurückbewegen in die Ruhestellung aus der Vertiefung (21) herausbewegbar ist, wobei die Vertiefung (21) in einer Richtung quer zur Trennrichtung (D2) durch zwei schräg zueinander ausgerichtete Begrenzungskanten (22) begrenzt ist, wobei das Betätigungselement (19) so ausgebildet ist, dass ein in die Vertiefung (21) eingreifender Abschnitt des Betätigungselements (19) beim Verlagern des Koppelabschnitts (8) in der Trennrichtung (D2) auf die Begrenzungskanten (22) zubewegt wird und - wenn der Koppelabschnitt (8) die Zwischenstellung erreicht - beidseitig an den Begrenzungskanten (22) anschlägt, sodass ein weiteres Verlagern des Koppelabschnitts (8) über die Zwischenstellung hinaus nicht möglich ist.

5. Atemmaske (1) nach einem der vorhergehenden Ansprüche,
wobei das Betätigungselement (19) als eine Betätigungslasche (19) ausgebildet ist, wobei die Betätigungslasche (19) an einem ihrer Enden mit dem Maskenkörper (2) und/oder dem jeweiligen Koppelelement (7a, 7b) verbunden ist und durch elastisches Verbiegen ihres anderen Endes in die Betätigungsstellung bewegbar ist, sodass auf die Betätigungslasche (19) in der Betätigungsstellung eine Federkraft zum Zurückstellen der Betätigungslasche (19) in die Ruhestellung einwirkt.

6. Atemmaske (1) nach einem der vorhergehenden Ansprüche,
wobei das Verriegelungselement (18) als eine Verriegelungslasche (18) ausgebildet ist, wobei die Verriegelungslasche (18) an einem ihrer Enden mit dem Maskenkörper (2) und/oder dem jeweiligen Koppelelement (7a, 7b) verbunden ist und durch elastisches Verbiegen ihres anderen Endes in die Entriegelungsstellung bewegbar ist, sodass auf die Verriegelungslasche (18) in der Entriegelungsstellung eine Federkraft zum Zurückstellen der Verriegelungslasche (18) in die Verriegelungsstellung einwirkt.

7. Atemmaske (1) nach einem der vorhergehenden Ansprüche,
wobei der Koppelabschnitt (8) von mindestens einem der Koppelelemente (7a, 7b) über einen Biegeabschnitt (50) mit einem übrigen Längsabschnitt (36) des jeweiligen Koppelelements (7a, 7b) verbunden ist, wobei der Biegeabschnitt (50) im Vergleich zum Koppelabschnitt (8) und zum übrigen Längsabschnitt (36) besonders biegsam ausgebildet ist, um ein Abwinkeln des Koppelabschnitts (8) relativ zum übrigen Längsabschnitt (36) zu ermöglichen.

8. Atemmaske (1) nach einem der vorhergehenden Ansprüche,
wobei die Kopfbänderung (5) ein erstes Band (6a) und ein zweites Band (6b) zum Spannen um den Kopf (K) des Patienten umfasst;
wobei jedes Koppelelement (7a, 7b) ferner eine erste Bandaufnahme (9a) zum Befestigen des ersten Bands (6a) und eine zweite Bandaufnahme (9b) zum Befestigen des zweiten Bands (6b) umfasst und so ausgebildet ist, dass - wenn der Maskenkörper (2) mittels der Kopfbänderung (5) gegenüber dem Gesicht des Patienten fixiert ist - die erste Bandaufnahme (9a) in Richtung einer Sagittalachse (H) des Patienten betrachtet zumindest teilweise zwischen dem Kinn und einem Ohr des Patienten und in Richtung einer zur Sagittalachse (H) orthogonalen Longitudinalachse (V) des Patienten betrachtet zumindest teilweise zwischen dem Kinn und der Schläfenregion des Patienten angeordnet ist und die zweite Bandaufnahme (9b) zumindest teilweise gegenüber der Schläfenregion des Patienten angeordnet ist.

9. Atemmaske (1) nach Anspruch 8,
wobei jedes Koppelelement (7a, 7b) so ausgebildet ist, dass - wenn der Maskenkörper (2) mittels der Kopfbänderung (5) gegenüber dem Gesicht des Patienten fixiert ist - die erste Bandaufnahme (9a) zumindest teilweise gegenüber einem Teil des Unterkiefers des Patienten, insbesondere gegenüber einem Unterkieferast, angeordnet ist.

10. Atemmaske (1) nach Anspruch 8 oder 9,
wobei jedes Koppelelement (7a, 7b) so ausgebildet ist, dass - wenn der Maskenkörper (2) mittels der Kopfbänderung (5) gegenüber dem Gesicht des Patienten fixiert ist - der mit der jeweiligen Koppelaufnahme (3a, 3b) gekoppelte Koppelabschnitt (8) in Richtung der Longitudinalachse (V) betrachtet zumindest teilweise zwischen der ersten Bandaufnahme (9a) und der zweiten Bandaufnahme (9b) angeordnet ist und/oder die zweite Bandaufnahme (9b) in Richtung der Sagittalachse (H) betrachtet zumindest teilweise zwischen der ersten Bandaufnahme (9a) und dem mit der jeweiligen Koppelaufnahme (3a, 3b) gekoppelten Koppelabschnitt (8) angeordnet ist.

11. Atemmaske (1) nach einem der Ansprüche 8 bis 10,
wobei - wenn der Maskenkörper (2) mittels der Kopfbänderung (5) gegenüber dem Gesicht des Patienten fixiert ist - das erste Band (6a) eine erste Zugkraft (F1) auf die erste Bandaufnahme (9a) eines jeden Koppelelements (7a, 7b) ausübt und das zweite Band (6b) eine zweite Zugkraft (F2) auf die zweite Bandaufnahme (9b) eines jeden Koppelelements (7a, 7b) ausübt, wobei eine in Richtung der Sagittalachse (H) wirkende Komponente der ersten Zugkraft (F1) größer als, vorzugsweise mindestens doppelt so groß wie eine in Richtung der Longitudinalachse (V) wirkende Komponente der ersten Zugkraft (F1) ist und/oder eine in Richtung der Sagittalachse (H) wirkende Komponente der zweiten Zugkraft (F2) größer als, vorzugsweise mindestens doppelt so groß wie eine in Richtung der Longitudinalachse (V) wirkende Komponente der zweiten Zugkraft (F2) ist.

12. Atemmaske (1) nach einem der Ansprüche 8 bis 11,
wobei jedes Koppelelement (7a, 7b) einen ersten Arm (10a) und einen zweiten Arm (10b) umfasst, wobei der erste Arm (10a) und der zweite Arm (10b) L- oder V-förmig miteinander verbunden sind und in einem Übergangsabschnitt (11) ineinander übergehen, wobei die erste Bandaufnahme (9a) zumindest teilweise im Übergangsabschnitt (11) angeordnet ist, wobei die zweite Bandaufnahme (9b) an einem dem Übergangsabschnitt (11) abgewandten Ende des zweiten Arms (10b) angeordnet ist, wobei der Koppelabschnitt (8) an einem dem Übergangsabschnitt (11) abgewandten Ende des ersten Arms (10a) angeordnet ist.

13. Atemmaske (1) nach einem der Ansprüche 8 bis 12,
wobei jede erste Bandaufnahme (9a) und/oder jede zweite Bandaufnahme (9b) eine im Material des jeweiligen Koppelelements (7a, 7b) ausgebildete Aufnahmeöffnung (12) zum Einführen des jeweiligen Bands (6a, 6b) umfasst, wobei die Aufnahmeöffnung (12) zumindest teilweise durch einen in einer Umfangsrichtung der Aufnahmeöffnung (12) gekrümmten Randabschnitt (13) begrenzt ist, wobei das an der jeweiligen Bandaufnahme (9a, 9b) befestigte Band (6a, 6b) den Randabschnitt (13) zumindest teilweise umschlingt, wobei eine Ausrichtung einer Längsachse des jeweiligen Bands (6a, 6b) relativ zur Sagittalachse (H) durch Verschieben des den Randabschnitt (13) umschlingenden Teils des jeweiligen Bands (6a, 6b) entlang des Randabschnitts (13) veränderbar ist.
